(19)

Europäisches
Patentamt

European
Patent Office

Office européen
des brevets

(11) **EP 4 668 780 A1**

(12) **EUROPEAN PATENT APPLICATION**

(43) Date of publication:
**24.12.2025 Bulletin 2025/52**

(21) Application number: **25179875.7**

(22) Date of filing: **30.05.2025**

(51) International Patent Classification (IPC):
*H04R 25/00* (2006.01)   *A61F 11/04* (2006.01)
*G09B 19/04* (2006.01)   *G09B 21/00* (2006.01)
*G10L 21/06* (2013.01)   *G10L 21/16* (2013.01)

(52) Cooperative Patent Classification (CPC):
**H04R 25/554; A61F 11/045; G09B 19/04;
G09B 21/009; G10L 21/06; G10L 21/16;
H04R 25/305; H04R 25/353; H04R 25/43;
H04R 25/453; H04R 25/606; H04R 2225/43;
H04R 2225/55; H04R 2225/61; H04R 2460/13**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **17.06.2024 EP 24182527**

(71) Applicant: **Oticon A/S
2765 Smørum (DK)**

(72) Inventors:
• **FAULKNER, Kathleen
  DK-2765 Smørum (DK)**

• **HARVEY, Ross
  DK-2765 Smørum (DK)**
• **INNES-BROWN, Hamish
  DK-2765 Smørum (DK)**
• **NAVNTOFT, Charlotte Amalie
  DK-2765 Smørum (DK)**
• **PEDERSEN, Rasmus Lundby
  DK-2765 Smørum (DK)**
• **SCHULTE, Alina
  DK-2765 Smørum (DK)**
• **SØRENSEN, Anna Josefine Munch
  DK-2765 Smørum (DK)**

(74) Representative: **Demant
Demant A/S
Kongebakken 9
2765 Smørum (DK)**

(54) **A HEARING AID SYSTEM COMPRISING A HAPTIC UNIT**

(57) In an aspect of the present disclosure, a hearing aid system comprises an input unit. The input unit comprises at least one microphone. The at least one microphone is configured to pick up audio from a sound environment. The input unit is configured to is configured to provide an audio input signal based on the audio. The hearing aid system comprises a signal processor. The signal processor is configured to receive the audio input signal and to provide, based on the audio input signal, a processed audio signal indicative of the audio. The signal processor is configured to provide, based on the audio input signal, a speech encoded signal. The speech encoded signal is indicative of speech characteristics in the audio. The hearing aid system comprises an output unit. The output unit is configured to receive the processed audio signal. The output unit is configured to provide, based on the processed audio signal, an auditory output sound. The auditory output sound is indicative of the sound environment. The hearing aid system comprises a haptic unit. The haptic unit is configured to receive the speech encoded signal. The haptic unit is configured to provide, based on the speech encoded signal, a haptic stimulation. The haptic stimulation is indicative of speech in the sound environment.

FIG. 1

**Description**

TECHNICAL FIELD

The present application generally relates to the field of hearing aid systems, haptic units, and speech intelligibility improvements.

BACKGROUND

Despite notable advancements in hearing aid (e.g., hearing instrument) research and development, hearing impaired users still struggle to distinguish speech from background noise in noisy environments, making it challenging for users to engage in conversations. Hearing aid companies have been exploring a number of technologies to improve the audio that is delivered to the receiver in the user's ear, or replacing the receiver with a bone anchored transducer. The solution in the present application is to approach this challenge from a new angle.

Another area of research and product development in the last few years has been bi-modal stimulation; the application of stimulation to multiple senses. Research conducted by these companies and others indicates that a multisensory integration can increase cognitive response compared to one sense alone. Significant improvements for patients have been shown in areas such as tinnitus control.

In particular, the focus may be on the haptic / tactile input. There have recently been a number of studies showing positive effects from haptic stimulation in various aspects of hearing or conversation.

In parallel, the boom in smartwatches in recent years means that there is a significant body of hearing aid users, wearing a haptic-capable device which is waiting to be enabled for our use cases. There is no stigma associated with a smart watch or similar, which is traditionally a barrier to adoption of new devices in the technical field.

SUMMARY

A hearing aid system:

In an aspect of the present disclosure, a hearing aid system comprises an input unit. The input unit comprises at least one microphone. The at least one microphone is configured to pick up audio from a sound environment. The input unit is configured to is configured to provide an audio input signal based on the audio.

The hearing aid system comprises a signal processor. The signal processor is configured to receive the audio input signal and to provide, based on the audio input signal, a processed audio signal indicative of the audio. The signal processor is configured to provide, based on the audio input signal, a speech encoded signal. The speech encoded signal is indicative of speech characteristics in the audio.

The hearing aid system comprises an output unit. The output unit is configured to receive the processed audio signal. The output unit is configured to provide, based on the processed audio signal, an auditory output sound. The auditory output sound is indicative of the sound environment.

The hearing aid system comprises a haptic unit. The haptic unit is configured to receive the speech encoded signal. The haptic unit is configured to provide, based on the speech encoded signal, a haptic stimulation. The haptic stimulation is indicative of speech in the sound environment.

An advantage of the present disclosure is a hearing aid system with an improved speech perception for a hearing aid user. This advantage can be achieved by providing an auditory output sound along with a haptic stimulation. In particular, the auditory output sound may contain speech, and the haptic stimulation may be indicative of a speech characteristic. Thereby, the hearing aid user is presented with closely related bi-modal stimulation which improves the speech perception. Moreover, the haptic device can provide tactile clues of speech occurring around a hearing aid user. This can allow users to selectively focus on important auditory cues while filtering out less relevant noise, thus potentially reducing auditory fatigue. For example, the disclosure may allow for easier conversation dynamics for the hearing aid user by providing tactile information about speech. As an example, the disclosure can enhance cues that make speech more predictable and make a hearing aid user able to synchronize their turn-taking to alleviate the cognitive load on them.

The improved speech intelligibility, as a result of an embodiment of the present disclosure, may be one step to facilitate smoother conversation, as improved speech intelligibility may enhance the lexicosyntactic turn-end cues and provides more context, thus leading to potentially less cognitive load on the individual to infer meaning from context.

Another step is to improve acoustic and prosodic cues to enhance turn-end prediction, is to enhance the perceived changes in pitch and loudness in speech, as pitch and loudness are good indicators of turn-ends across languages. Thus, these cues are deemed relevant and feasible to enhance with haptic stimulation. The pitch slope, duration, and range, constituting the speech encoded signal, have been found to be turn-end predictors. The pitch is the human percept of the physical quantity F0, or the fundamental frequency. To convert the pitch properties into haptic stimulations, one would have to extract the F0 of the desired speaker's speech. A drop in loudness at the end of an utterance is also predictive of a turn-end, and the loudness may be be converted into a speech intensity signal used to provide the haptic stimulation. A final way to enhance the predictability of the desired speaker's speech is by sending a haptic signal at the syllable rate to increase the predictability of when to take a turn in conversation.

The hearing aid system may comprise an input unit for providing an audio input signal. The input unit may comprise an input transducer, e.g. a microphone. The input unit may be configured to pick-up audio from an input transducer. The picked-up audio being indicative of the sound environment. The input unit may comprise a wireless receiver for receiving a wireless signal comprising the picked-up audio by an auxiliary device.

The audio input signal may be represented as a time domain signal wherein a time domain signal may be defined as a signal with a time-varying amplitude. An audio input signal may be represented as a frequency domain signal wherein a frequency domain signal may be defined as a signal with a frequency-varying amplitude and/or phase. An audio input signal may be represented as a time-frequency domain signal, wherein a time-frequency domain signal may be defined as a signal with a time-varying and frequency-varying amplitude and/or phase.

In an example embodiment, the one or more audio input signals are represented in the time domain.

An audio input signal to the input unit may be an analog signal represented as a continuous signal in time and amplitude. The audio input signal may be converted into a digital signal represented as a discrete signal in time and amplitude. Hence, a discrete signal may be characterized by a finite time and amplitude resolution. The conversion from an analog signal to a digital signal may be performed by an analog-to-digital converter (ADC). The ADC may be configured to have a pre-defined amplitude and time resolution given a bit-resolution and a sampling frequency, respectively. The hearing aid system may comprise the ADC. The hearing aid system may comprise a plurality of ADCs, so that each ADC is assigned to each audio input signal.

The audio input signal may be based on a picked-up audio by a microphone or a transducer sensitive to acoustic vibrations. The microphone can be understood as a transducer configured to convert acoustic energy (e.g., audio) to electrical signals.

The audio input signal may be based on a picked-up accelerometer signal by an accelerometer indicative of an acceleration or a movement. The audio input signal may be based on an EEG signal or EOG signal picked-up by electrodes, wherein the EEG signal or EOG signal is indicative of an electrical activity of the brain. The audio input signals may be acquired by different types of transducers. For example, a first audio input signal, picked-up by a microphone, may be indicative of sound and a second audio input signal, and a second audio input signal, picked up by an accelerometer, may be indicative of movement.

A sound environment may refer to the collection of audible sound sources within an area or space and may include reflections of sound such as reverberation and echo. A sound environment may be considered an area around a use of the hearing aid system. An audible sound source may be a person speaking, a loudspeaker, or any element able to provide an audio. An audio may be characterized by a sound type which may include speech, music, alarms, tones, music, noise, etc.

Audio input signal may be a pre-processed. For example, the audio input signal may be a time domain signal and pre-processed by a filter bank to transform the audio input signal into the time-frequency domain such that the audio input signal is a time-frequency domain signal. The input unit may be configured to provide a plurality of audio input signals if the input unit receives a plurality of different types of audio input signals, e.g. from an accelerometer and a microphone. Thus, the audio input signal may constitute a plurality of audio input signals. In certain examples, the input unit is configured to convert the audio to the audio input signal.

The signal processor can be configured to receive and process the audio input signal. The signal processor may be configured to the audio input signals. The signal processor may be configured to provide a processed audio signal based on the audio input signal. The processed audio signal can be indicative of the audio. The signal processor may comprise an analysis filter bank configured to transform the audio input signal into the time-frequency domain. The hearing aid system may comprise the memory unit.

The signal processor may be a computer chip constituting the hearing aid system. The signal processor may be a part of the computer chip.

A processed audio signal may be based on one or more of the audio input signals. The signal processor may be configured to provide a plurality of processed audio signals. The signal processor may be configured to provide the processed audio signal by processing the audio input signals. For example, the signal processor may comprise one or more of following: a beamformer, a single-channel filter, a neural network trained to extract speech from the audio input signals, a hearing loss compensation algorithm, a feedback cancellation system.

The signal processor may comprise a noise reduction system configured to attenuate noise in the audio input signal. For example, the noise reduction system can be configured to attenuate noise in the audio input signal so that the sound quality and/or speech intelligibility may be perceived as improved compared to the one or more audio input signals by using, e.g., a beamformer, a single-channel filter, a neural network trained to extract speech from the audio input signals, etc.

The processed audio signal may be characterized in that it contains less background noise compared to the audio input signals by the noise reduction system.

The signal processor may be configured to provide a plurality of processed audio signals. For example, a hearing aid system comprising two hearing aids, the signal processor may be configured to provide two processed audio signals, i.e., one for each hearing aid. The signal processor may comprise a synthesis filter bank configured to transform the processed audio signals into the time-frequency domain.

A speech encoded signal may be understood as a signal comprising speech features or speech information or speech cues. The speech features or speech information or speech cues can each be indicative of a speech characteristic and obtained based on the audio input signal. For example, the speech encoded signal may comprise speech features obtained based on the audio input signals.

The articulation of speech may be a speech characteristic. The pronunciation of of speech may be speech characteristic. The speech activity may be a speech characteristic. The speech pitch may be a speech characteristic. The speech rate may be a speech characteristic. The speech rhythm may be a speech characteristic. The speech tone may be a speech characteristic. The speech characteristic may be determined by a voice activity detector determining the speech activity. The speech characteristic may be determined by a spectrum analyzer determining the speech pitch, the speech tone, etc. The speech characteristic may be determined by a modulation analyzer.

The signal processor may be configured to provide a speech encoded signal. The signal processor may be configured to provide a plurality of speech encoded signals. Each of the speech encoded signals may comprise speech characteristics for different speech for different speakers.

An output unit may be configured to receive the processed audio signals. The output unit may be configured to provide, based on the processed audio signal, an auditory output signal. The auditory output signal can be based on the processed audio signal. An output signal may be represented as a time domain signal. An output signal may be represented as a frequency domain signal. An output signal may be represented as a time-frequency domain signal.

The output unit may comprise an output transducer. The output transducer may be a hearing aid receiver. The output transducer may be a loudspeaker. The output unit may comprise an amplifier configured to amplify the processed audio signal to a desired sound pressure level or a desired gain characteristics. The output unit may be configured to provide an auditory output sound. The auditory output sound may be a sound heard by a hearing aid user as provided by the hearing aid system. The auditory output signal can be indicative of the sound environment.

The output unit may receive the processed audio signal as a digital signal. The output unit may be configured convert processed audio signal into an analog signal. The conversion from a digital signal to an analog signal may be performed by a digital-to-analog converter (DAC). The DAC may be configured to receive a digital signal with a pre-defined amplitude and time resolution. The hearing aid system may comprise the DAC. The hearing aid system may comprise a plurality of DACs, so that each DAC is assigned to each processed audio signals.

The output unit may comprise a plurality of output transducers. For example, a hearing aid system may comprise two hearing aids. The hearing aid system may provide a processed audio signal to each output unit. The output unit may comprise an output transducer for each hearing aid. Each output transducer may provide an auditory output sound.

In a binaural hearing aid system, the hearing aid system may comprise two hearing aids. Each hearing aid may comprise an input unit, a signal processor, and an output unit.

A haptic unit may comprise a haptic transducer. The haptic transducer may be considered as an output transducer that provides, based on the speech encoded signal, haptic sensations (e.g., physical sensations, haptics, tactile sensations, tactile feelings). The haptic sensation may be in the form of vibrations. The haptic sensations may be in the form of forces that can be felt by the hearing aid user. The haptic sensations may be electrical stimulations.

In other words, the haptic unit may be used to create haptic feedback providing tactile information to the hearing aid user about the speech encoded signal. The haptic transducer may be based on an actuator configured to generate motion or force. The actuator may include a piezoelectric actuator, an electromagnetic actuator, an electroactive polymer, eccentric rotating mass actuators, etc.

A haptic stimulation may refer to the process of delivering the physical or tactile sensation to the hearing aid user through physical interaction with the haptic transducer. The haptic stimulation may be perceived as a sense of touch which may be felt on the skin of the hearing aid user or through muscles. The sensation of the haptic stimulation may include vibrations, pressure, texture, and motion.

The speech encoded signal may comprise a speech envelope signal. The speech encoded signal may comprise a speech timing signal. The speech encoded signal may comprise a speech intensity signal. The speech encoded signal may comprise a combination of the any of the aforementioned speech encoded signals.

An advantage of the present disclosure is that the haptic stimulation is based on a speech characteristic instead of the processed audio signal, where the processed audio signal may contain cues that are not relevant for haptic stimulation. Thereby, the haptic stimulation can be based on relevant speech characteristics for haptic presentation in order to enhance the speech perception.

A speech envelope signal may be understood as the envelope of a speech signal constituting the audio input signal. An envelope may refer to a smooth curve that outlines the extremes of a signal. The envelope may represent the instantaneous amplitude the signal time. A speech signal may refer to a signal with a time-varying amplitude (or spectrum) representing a speech sound.

The speech envelope signal may be determined based on the processed audio signal. The speech envelope signal may be determined based on an analytic signal, wherein the analytic signal is based on the audio input signal. The analytic signal may be defined based on the Hilbert transform. The speech envelope signal may be determined based on average power of audio input signal in time-segments or frames, e.g. by means of the root-mean-square (RMS) value. The speech envelope signal may be determined based on the processed audio signal.

A speech timing signal may be the onset of a speech signal constituting the audio input signal. The speech timing signal may be the onset of a speech phoneme or a speech syllable or a spoken word of a speech signal. The speech timing signal may be the offset of a speech phoneme or a speech syllable or a speech syllable or a spoken word of a speech signal. The onset may be defined as a timestamp wherein the energy of the speech signal exceeds a pre-defined or adaptive threshold. The offset may be defined as a timestamp wherein the energy of the speech signal is below to a pre-defined or adaptive threshold. The speech timing signal may be duration of a speech phoneme or a speech syllable or a spoken word of a speech signal. The speech timing signal may be a plurality of onsets and/or offsets or durations based on a speech signal. The speech timing signal may be determined based on the processed audio signal.

A speech intensity signal may be the intensity or loudness of a speech signal constituting the audio input signal over time. The intensity may be the power or the energy of the speech signal. The intensity may be the dynamic range of the speech signal. The speech intensity signal may be determined based on the processed audio signal. The speech intensity signal may be a spectrum or part of a spectrum of the speech signal. The part of the spectrum of the speech signal may be a fundamental frequency such as an F0-component of speech or a pitch.

The speech signal may be based on the processed audio signal.

The speech encoded signal may comprise a peak of the speech envelope. The peak may be considered maximum value of the speech envelope within a pre-defined amount of time. The speech encoded signal may comprise a plurality of peaks of the speech envelope. Each peak may be considered a local maximum value of the speech envelope within a pre-defined amount of time. A local maximum may be considered as a maximum value within a second amount of time.

The speech encoded signal may be a combination of one or more of following: the speech envelope signal, the speech timing signal, and the speech intensity signal. The combination may be determined as a linear combination between at least two of the speech envelope signal, the speech timing signal, and the speech intensity signal, wherein the weights may be pre-determined by a hearing care processional configuring the hearing aid system or a developer using an embodiment of the present disclosure.

The signal processor may comprise a speech enhancer. The speech enhancer may be configured to provide, based on the audio input signal, a speech enhanced signal. The speech encoded signal may be based on the speech enhanced signal.

An advantage of the present disclosure is that the processed audio signal and the haptic stimulation is based on the output of a speech enhancer which reduces background noise. Thereby, the task of extracting speech characteristics becomes easier since the speech enhanced signal contains less noise that may corrupt the determination of the speech encoded signal.

A speech enhancer may be configured to receive one or more audio input signals. The speech enhancer may be configured to receive one or more pre-processed audio input signals. The pre-processed audio input signals may include frequency-shaping or filtering of the audio input signal. The speech enhancer may comprise a beamformer. For example, the speech enhancer may include the beamformer if the speech enhancer receives a plurality of audio input signal or pre-processed audio input signals.

The beamformer (e.g., a delay-and-sum beamformer, a minimum variance distortion-less response (MVDR) beamformer, and a multichannel Wiener filter (MWF) beamformer) may be configured to combine the audio input signals to provide the processed audio signal. The beamformer may use beamformer weights. The beamformer weights may be used to form a linear combination of beamformer input signals. The beamformer input signals may be the audio input signals. The beamformer weight may be complex numbers. The beamformer weights used by the beamformer may be pre-determined such that the beamformer has a pre-defined beampattern. The beamformer weights may be determined adaptively.

The speech enhancer may comprise a single-channel filter. The single-channel filter may be configured to modify the frequency characteristics of the audio input signals or pre-processed audio input signals. The speech enhancer may comprise a post-filter. The post-filter may be configured to modify the frequency characteristics of the beamformer output to reducing background noise by attenuating frequency bands dominated by noise. The speech enhancer may comprise a trained neural network configured to extract a desired speech signal. The trained neural network may be trained off-line on audio input signals representative of a sound environment and a target speech signal.

The speech enhancer may comprise a source separation method based on one or more of a beamformer, a single-channel filter, a neural network, and a blind source separation method. The speech enhancer may be based on a method improving the speech intelligibility and/or speech quality of the audio input signal. The speech enhancer may comprise an echo canceller and/or a dereverberation method configured to reduce echo and/or reverberation in the sound environment.

A speech enhanced signal may be the output of the extracted desired speech signal. The speech enhanced signal may be represented in the time domain, the frequency domain, or the time-frequency domain. The speech enhanced signal has an improved signal-to-noise ratio compared to the audio input signal.

The signal processor may comprise a downsampler. The downsampler may be configured to reduce a data bandwidth of the speech encoded signal. The downsampler may be configured to provide a downsampled speech encoded signal. The speech encoded signal may be based on the downsampled speech encoded signal.

An advantage of the present disclosure is a reduced power consumption since the downsampled speech encoded signal has a lower data bandwidth than the speech encoded signal before downsampling. Further, the system does not need to send the whole speech encoded signal to the haptic unit.

A downsampler may be regarded as a system configured to reduce a bit-rate and/or bit-resolution of the speech encoded signal compared to the bit-rate and/or bit-resolution of the audio input signal. The downsampler may comprise a low-pass filter configured to reduce the frequency bandwidth of the speech encoded signal. The downsampler may comprise a data compressor. The data compressor may be configured to reduce the sample-rate of the speech encoded signal.

A data bandwidth may comprise the bit-rate. The data bandwidth may comprise bit-resolution. The data bandwidth may comprise sample-rate of a signal. The data bandwidth may be a combination of the bit-rate, the bit-resolution, and the sample-rate.

The signal processor may comprise a speech quality estimator. The speech quality estimator may be configured to receive a speech quality estimator input signal. The speech quality estimator input signal may be based on the audio input signal. The speech quality estimator may be configured to provide a speech quality value. The speech quality value may be indicative of a speech quality. The signal processor may be configured to receive the speech quality value. The signal processor may be configured to operate in a first mode or a second mode of operation. The signal processor may be configured to switch from a first mode of operation to a second mode of operation if the speech quality value is below a first threshold. The signal processor may be configured to not switch from a first mode of operation to a second mode of operation if the speech quality value is equal to or above a first threshold. The signal processor, in the first mode of operation, may be configured to provide the speech encoded signal. The signal processor, in the second mode of operation, may be configured to not provide the speech encoded signal.

An advantage of the present disclosure is that haptic stimulation may only be presented to the hearing aid user when the speech quality is above a threshold and thereby improving the user experience of the haptic stimulation.

A speech quality estimator may be configured to estimate a speech quality of the speech quality estimator input signal. The speech quality estimator input signal may be the audio input signal. The speech quality estimator input signal may be the speech enhanced signal. The speech quality estimator input signal may be the processed audio signal. The speech quality estimator may be based on an algorithm configured to determine a speech quality value, wherein the speech quality value is indicative of speech quality and/or speech intelligibility and/or speech activity.

The speech quality value may be a value defined between (and including) '0' and '1', where a value of '0' may be indicative of poor or low speech quality or speech intelligibility or speech activity and a value of '1' may be indicative of a good or high speech quality or speech intelligibility or speech activity. Alternatively, a value of '1' may be indicative of poor or low speech quality or speech intelligibility and a value of '0' may be indicative of a good or high speech quality or speech intelligibility or speech activity. Alternatively, the numerical range of the speech quality value may be unbounded. The speech quality value may include on a short-term objective intelligibility (STOI) score, a perceptual evaluation of speech quality (PESQ) score, a signal-to-noise ratio (SNR) value, a speech presence probability, an own voice activity value, a noise level, etc.

The speech quality estimator may provide the speech quality value for each frequency band. The speech quality estimator may provide a global speech quality value indicative of an average speech quality across frequency bands. The speech quality estimator may provide the speech quality value for a time-segment of the audio input signal.

The signal processor may operate in two modes of operation. In the first mode of operation, the hearing aid may be configured to provide the the speech encoded signal. In the second mode of operation, the hearing aid may be configured to not provide the speech encoded signal. For example, the speech encoded signal may comprises only values of '0' indicative of no speech characteristics may encompass not providing the speech encoded signal. The signal processor may comprise a conditional statement to determine the mode of operation. Alternatively, in the first mode of operation, the hearing aid may be configured to not provide the the speech encoded signal. In the second mode of operation, the hearing aid may be configured to provide the speech encoded signal.

The signal processor may be configured to operate in two modes, wherein in one mode the signal processor is configured to provide the speech encoded signal, and the not provide the speech encoded signal in the other mode.

A first threshold may comprise a value in the numerical range of the speech quality value. For example, if the numerical range of the speech quality value is between '0' and '1', then the first threshold may also be a value between '0' and '1'. The first threshold may be predetermined by a hearing care professional or a developer. The first threshold may be determined adaptively based on an analysis of one or more of the speech quality value. For example, the first threshold may be determined adaptively by analyzing the signal-to-noise ratio. For example, the analysis may determine the dynamic range of the signal-to-noise ratio. The first threshold may be determined based on the dynamic range of the signal-to-noise ratio. If the dynamic range is high, the first threshold may be set to be a value closer to '1'. If the dynamic range is low, the first threshold may be set to a value closer to '0'. The first threshold may be set to a value of 0.5. The first threshold may be set to a value of 0.9. The first threshold may be set to a value of 0.1. The first threshold may be set to a value of in the range 0.05 to 0.95.

The signal processor may switch from the first mode of operation to the second mode of operation if the speech quality value is below the first threshold. The signal processor may switch from the second mode of operation to the first mode of operation if the speech quality value is above or equal to the first threshold.

The haptic unit may be configured to receive the speech quality value.

The speech quality value may be indicative of a signal-to-noise ratio. The speech quality value may be indicative of a noise level. The speech quality value may be indicative of a speech presence probability. The speech quality value may be indicative of an own voice activity value. The speech quality value may be indicative of a combination of the aforementioned speech quality values.

An advantage of the present disclosure is that the speech quality value is based on a parameter that is a readily available on a hearing aid system and thereby saving computational complexity which may lead to more compact hearing aids due to smaller computer chip size, lower cost of production hearing aids due to lower complexity computer chips, and reduced power consumption due to lower complexity computer chips.

A signal-to-noise ratio may be defined as the ratio or difference between the signal energy of the desired speech signal and the signal energy of the background noise. The signal-to-noise ratio may be determined by the speech enhancer. For example, the speech enhancer may comprise a noise reduction system comprise a single-channel filter using an estimated signal-to-noise ratio, wherein the speech enhancer may further comprise a signal-to-noise ratio estimator, wherein the signal-to-noise may be estimated based on the audio input signal. Thereby, the speech quality value may use the estimated signal-to-noise ratio by the speech enhancer.

In an example embodiment, the speech quality value may be a signal-to-noise ratio in decibels (dB). The first threshold may be a value of 6 dB or 8 dB or 10 dB or any value in the range of 0 dB to 20 dB. If the signal processor is in the first mode of operation and the speech quality value is above the first threshold, then the signal processor remains in the first mode of operation. If the signal processor is in the first mode of operation and the speech quality value is below the first threshold, then the signal processor switches to the second mode of operation. If the signal processor is in the second mode of operation and the speech quality value is above the first threshold, then the signal processor switches to the first mode of operation. If the signal processor is in the second mode of operation and the speech quality value is below or equal to the first threshold, then the signal processor remains in the second mode of operation.

The speech quality estimator may comprise a sigmoid function to map a speech quality value into a value between (and including) '0' and '1'. The speech quality estimator may comprise a piecewise linear function to map a speech quality value into a value between (and including) '0' and '1'.

A noise level may be defined as the signal energy of the background noise. The noise level may be determined by the speech enhancer comprising a noise level estimator. For example, the noise level estimator may be determined by tracking the noise floor of the audio input signal.

A speech presence probability may be defined as the probability of which speech is present in the audio input signal or the speech enhanced signal or the processed audio signal. The speech presence probability may be determined by the speech enhancer comprising a speech presence probability estimator. The speech presence probability estimator may estimate the speech presence probability based on a statistical model of the audio input signal.

An own voice activity value may be defined as the likelihood of which the hearing aid user's own voice is present in the audio input signal or the speech enhanced signal or the processed audio signal. The own voice activity value may be determined by the speech enhancer comprising an own voice activity estimator. The own voice activity value may be determined based on an own voice beamformer and analyzing the modulation index of the output of the own voice beamformer, wherein a high modulation index may be indicative of own voice activity. The own voice activity value may be an own voice likelihood value.

In an example embodiment, the signal processor may comprise an own voice activity estimator. The own voice activity estimator may be configured to receive an own voice activity estimator input signal based on the audio input signal. The own voice activity estimator may provide the own-voice likelihood value indicative of the probability that a speech signal originates from the hearing aid user's own voice. The signal processor may be configured to receive the own-voice likelihood value and switch from a first mode of operation to a second mode of operation if the own-voice likelihood value is above a first threshold. The signal processor may in the first mode of operation be configured to provide the speech encoded signal. The signal processor may in the second mode of operation be configured to not provide the speech encoded signal.

The speech quality value may be a combination of the speech quality values. For example, the combination may be determined as a linear combination of the speech quality values using at least one speech quality combination weight which may be pre-determined by a hearing care professional or a developer.

The haptic unit may comprise a wave modulator. The wave modulator may be configured to receive a carrier signal and a modulator signal. The modulator signal may be based on the speech encoded signal. The wave modulator may be configured to modulate the carrier signal based on the modulator signal for provision of a modulated signal. The haptic stimulation may be based on the modulated signal.

An advantage of the present disclosure is that the haptic stimulation is based on a modulated signal which may improve the perceived speech intelligibility instead of presenting a haptic stimulation based on the speech encoded signal.

A wave modulator may be defined as a system that alters the one or more signal properties of a carrier signal based on the modulator signal. A carrier signal may be a waveform. A carrier signal may be a waveform that is used to modulate with the modulator signal. A carrier signal may be a waveform used to carry the modulator signal. The carrier signal may be a wave used to carry the speech encoded signal. A signal property may include an amplitude characteristic, a frequency characteristic, or a phase characteristic of the carrier signal. The wave modulator may be an amplitude modulator. The wave modulator may be a frequency modulator. The wave modulator may be a phase modulator. The wave modulator may be a pulse modulator.

A carrier signal may be a pre-defined signal. The haptic unit may comprise the predefined signal. The haptic unit may comprise a memory device configured to store the pre-defined signal.

The wave modulator may comprise a peak detector. The peak detector may be configured to detect peaks in the speech encoded signal. For example, if the speech encoded signal is a speech envelope, the peak detector may be configured to detect peaks in the speech envelope. The peak detector may be configured to provide, based on the speech encoded signal, a peak signal. The wave modulator may comprise a peak rate detector. The peak rate detector may be configured to determine the rate of change in the speech encoded signal. The rate of change may be determine based on determining the derivative of the speech encoded signal. The derivative may be determined based on determining the difference between neighboring values in the time-series constituting the speech encoded signal. For example, if the speech encoded signal is a speech envelope, the peak rate detector may be configured to provide, based on the speech envelope, a peak rate signal.

The modulator signal may be a peak signal. The modulator signal may be a peak rate signal.

A modulator signal may be the speech encoded signal.

A modulator signal may be the output of the wave modulator.

In an example embodiment, the wave modulator comprises a frequency modulator.

The hearing aid system may comprise a plurality of wave modulators. The speech encoded signal may comprise the speech characteristics of a plurality of speech signals. The haptic unit may receive a plurality of speech encoded signals. The haptic unit may be configured to provide a haptic stimulation for each speech encoded signal. The wave modulator may be configured to modulate a carrier signal based on each speech encoded signal for provision of a plurality of modulated signals. The haptic stimulation may be based on the plurality of modulated signals.

In an example embodiment, the wave modulator comprises a combination of a frequency modulator and an amplitude modulator. The wave modulator provides, based on the speech encoded signal, a frequency modulated signal using a frequency modulator followed by providing, based on the frequency modulated signal, a frequency-amplitude modulated signal using an amplitude modulator. The haptic stimulation may be based on the frequency-amplitude modulated signal.

The haptic unit may comprise a wavetable. The wavetable may comprise a plurality of carrier signals. The carrier signal may be based on a selected carrier signal from the plurality of carrier signals of the wavetable. The haptic unit may be configured to determine the selected carrier signal based on the speech encoded signal.

An advantage of the present disclosure is that the haptic stimulation is based on a known set of carrier signals that may be known to improve the perceived speech intelligibility.

A wavetable may comprise a sound synthesizer. The sound synthesizer may comprise a plurality of pre-defined waveforms. The sound synthesizer may comprise an oscillator configured to provide the waveform with a wave characteristic. A wave characteristic may include a frequency, an amplitude, an envelope, a shape of the wave. The wave characteristic may be time dependent, e.g., change over time.

A carrier signal may be the waveform provided by the wavetable. The waveform may include sine waves, square waves, saw waves, triangle waves, pulse wave, a wavelet, or a combination of the waveforms. The pulse wave may be an impulse signal. The envelope of the wavelet may be characterized by being bell-curve shaped.

The haptic unit may be configured to select the carrier signal from the plurality of carrier signals of the wavetable based on a wavetable setting. The wavetable setting may be determined by a hearing care professional, or a developer, or the hearing aid user based on a preference. The hearing aid user may determine the wavetable setting through an interface of an auxiliary device, or the haptic unit, or the hearing aid.

The haptic unit may be configured to select the carrier signal from the plurality of carrier signals of the wavetable based on the speech encoded signal. For example, the speech intensity value constituting the speech encoded signal may be used to determine the amplitude or the frequency of the waveform. For example, the spectrum constituting the speech intensity value may be used to determine the carrier signal. For example, if the speech signal, constituting the audio input signal, is perceived as a soft sound, the carrier signal may be selected as a carrier wave whose shape is rounded, e.g. a continuous wave. If the speech signal, constituting the audio input signal, is perceived as a hard sound, the carrier signal may be selected as a carrier wave whose shape is sharp, e.g. a discontinuous wave.

The soft sound may refer to sounds that are perceived as mellow and smooth. The soft sound may be characterized by a perceived lower amplitude, a more rounded sound, and a spectrum with more energy in the lower frequency bands compared to hard sound sounds. For example, vowels may be considered has a soft sound.

The hard sound may be perceived as the opposite of the soft sound. The hard sound may refer to sounds that are sharp, aggressive, and pronounced. The hard sound may be characterized by a higher amplitude, and a spectrum a spectrum with more energy in the higher frequency bands compared to soft sounds. The hard sounds may be perceived as intense and harsh.

The haptic unit may be configured to select the carrier signal from the plurality of carrier signals of the wavetable based on the speech quality value. For example, the speech quality value may be a signal-to-noise ratio. If the signal-to-noise ratio is below a second threshold, the selected carrier signal may be a pulse wave. If the signal-to-noise ratio is below a second threshold, the selected carrier signal may be a sine wave.

The haptic unit may be configured to select the carrier signal from the plurality of carrier signals of the wavetable based on the speech encoded signal and the speech quality value.

The haptic unit may, in the second mode of operation, not receive the speech encoded signal and provide no haptic stimulation.

**In** an example embodiment, the wave modulator comprises a frequency modulator and the selected carrier signal is a saw wave. The wave modulator is configured to receive speech encoded signal as the modulator signal and saw wave as the carrier signal.

The wave modulator is configured to frequency modulate the saw wave with the speech encoded signal and provide a frequency modulated signal. The speech encoded signal may be a speech envelope signal. The haptic stimulation may be based on the frequency modulated signal.

The input unit may be configured to receive a second input signal from an auxiliary device. The input unit may be configured to provide a second audio input signal.

An advantage of the present disclosure is that the hearing aid system may provide two types of audio input signals, and hence allowing the hearing aid user to listen to audio based on a sound environment and/or audio based on an auxiliary device.

The auxiliary device may be configured to provide a multimedia sound as the second input signal to the input unit. The multimedia sound may refer to audio that originates from various forms of media, such as music, video games, movies, animations, presentations, websites, and other interactive applications. The multimedia sound may be received through a network, e.g. a local network or a global network (e.g., the internet) or through a digital storage, e.g. a memory device on the auxiliary device.

The auxiliary device may comprise a remote microphone. The remote microphone may be configured to pick-up a second audio from the sound environment and provide the second input signal. The auxiliary device may be placed closer to the desired speaker to pick up the second audio comprising less noise compared to the audio picked-up by the at least one microphone of the hearing aid. The auxiliary device may comprise a second speech enhancer configured to provide, based on the second audio, a noise-attenuated second audio input signal. The input unit may receive the noise-attenuated second audio input signal.

The auxiliary device may be a television. The auxiliary device may be a cellular phone. The auxiliary device may be a device that is in wireless communication with the hearing aid system. The second audio input signal may be a streaming signal. The auxiliary device may be a watch such as a smart watch. The auxiliary device may be a band such as a smart band.

The hearing aid system may receive an input control signal. The input control signal may be used to select if the input unit provides the audio input signal based on the second input signal, received by the auxiliary device, or a first input signal received by the at least one microphone constituting the hearing aid. The input control signal may be determined by the hearing aid user through an interface on the hearing aid or the auxiliary device. The input control signal may be a binary value.

The hearing aid system may comprise a hearing aid and a haptic device. The hearing aid may comprise the input unit. The hearing aid may comprise the signal processor. The hearing aid may comprise the output unit. The haptic device may comprise the haptic unit.

An advantage of the present disclosure is that the hearing aid and haptic device may be two separate device and hence not limited to be located in the same local body part of the hearing aid user.

The hearing aid system may comprise two hearing aids and a haptic device. The hearing aid may each comprise an input unit, a signal processor, and an output unit. The two hearing aids may be configured to form a binaural hearing aid with a binaural communication link. The hearing aid system may select the hearing aid, of the two hearing aids with the best communication link to the haptic device, to provide the speech encoded signal. The best communication link may be determined based on a wireless signal-to-noise ratio. The best communication link may be determined as the hearing aid with the highest wireless signal-to-noise ratio to the haptic device. The selected hearing aid to communicate with the haptic device may be pre-determined by a hearing care professional, or a developer, or the hearing aid user.

The haptic device may be body-worn. The haptic device may be a wrist-worn haptic device placed at the wrist. The haptic device may be placed at the arm. The haptic device may be head-worn. The haptic device may be worn within the oral cavity. The haptic device may be a watch, for example a smart watch. The haptic device may be a band. The haptic device may be glove. The haptic device may be glasses, for example smart glasses. The haptic device may be a head-wear, for example a smart hat.

The hearing aid system may comprise a hearing aid comprising the input unit, and the signal processor, and the output unit, and the haptic unit. The hearing aid and the haptic unit may communicate by wire (e.g., directly connected).

The haptic device may constitute the first auxiliary device or a second auxiliary device. The second auxiliary device may be a body-worn device.

The hearing aid system may comprise a plurality of haptic devices. The haptic device may receive a speech encoded signal.

The hearing aid (HA) and the haptic device (HAPD) may be configured to wirelessly communicate.

An advantage of the present disclosure is that the hearing aid and haptic device are not limited to wired communication and may communicate through a wireless communication channel, thereby being more user friendly and convenient for the hearing aid user.

The hearing aid may comprise a first wireless communication system comprising an antenna. The antenna may be configured to transmit a wireless signal based on the speech encoded signal. The first communication system may be configured to receive a second wireless signal from the auxiliary device. The haptic unit may comprise a second wireless communication system comprising a second antenna. The second antenna may be configured to receive the wireless signal based on the speech encoded signal. The second wireless communication system may be configured to convert the wireless signal to the speech encoded signal.

The hearing aid system may be configured to determine a timing value. The hearing aid system may be configured to synchronize the provision of the haptic stimulation and auditory output sound. The synchronization may be based on the timing value.

An advantage of the present disclosure is that the presentation of the auditory output sound and the haptic stimulation are in sync, so that it is easier for a hearing aid user to identify or relate the haptic stimulation with the corresponding desired speech signal in the auditory output sound.

The hearing aid system may comprise a first timer configured to determine the timing value. The timing value may be indicative of a timestamp. The timing value may be indicative of a round-trip time between the hearing aid and the haptic device. The timing value may be indicative of a signal delay between the hearing aid and the haptic device. The hearing aid may comprise the first timer. The first wireless communication system may comprise the first timer. The haptic device may comprise a second timer. The second timer may be configured to determine a second timing value. The second timing value may be indicative of a second timestamp. The hearing aid system may be configured to determine a global timing value based on the first timing value, determined by the hearing aid, and the second timing value, determined by the haptic device. The global timing value may be a round-trip time between the hearing aid and the haptic device. The global timing value may be a signal delay between the hearing aid and the haptic device.

The haptic unit may be configured to compare the timing value with a timing threshold. The timing threshold may be pre-determined by the hearing care professional, or the developer, or the hearing aid user. The haptic unit may be configured to provide the haptic stimulation if the timing value is below or equal to the timing threshold. The haptic unit may be configured to not provide the haptic stimulation if the timing value is above the timing threshold.

The timing threshold may be a value within the range of 0 ms to 60 ms or 0 ms to 200 ms. The timing threshold may be 60 ms or below 60 ms. The timing threshold may be 200 ms or below 200 ms.

The hearing aid system may be configured to synchronize the provision of the auditory output sound and the haptic stimulation based on the timing value. Synchronizing the provision of the auditory output sound and the haptic stimulation may comprise delaying the haptic stimulation so that a desired bi-modal stimulation delay is achieved. The haptic device may be configured to delay the haptic stimulation. The desired bi-modal stimulation delay may be a value within the range of 0 ms to 60 ms or 0 ms to 200 ms. The desired bi-modal stimulation delay may be 15 ms. The desired bi-modal stimulation delay may be 30 ms. The desired bi-modal stimulation delay may be 50 ms.

For example, if the timing value is 10 ms, and the timing value is indicative of the signal delay between the hearing aid and the haptic device, and the desired bi-modal stimulation delay is 15 ms, then the haptic device may be configured to delay the provision of the haptic stimulation by 5 ms to achieve the desired bi-modal stimulation delay. Thereby, the provision of the auditory output sound and the haptic stimulation is synchronized when the desired bi-modal stimulation delay is achieved.

The signal processor may comprise a delay unit. The delay unit may be configured to apply a delay to the second audio input signal. The delay may be determined based on the timing value.

An advantage of the present disclosure is that the second audio input signal may be delayed so that the presentation of the haptic stimulation and the auditory output sound, based on the second audio input signal, are aligned in time, and thereby improving the speech perception.

A delay unit may be configured to delay the second audio input signal. The delay unit may be configured to filter the second audio input signal. The filter of the delay unit may be based on an all-pass filter. The filter of the delay unit may have a linear 0 dB magnitude or amplitude response and a linear phase response. The delay unit may delay the second audio input signal by an integer factor. The integer factor may be indicative of the sample delay. The delay unit may delay the second audio input signal by a non-integer factor by filtering the second audio input signal.

The hearing aid system comprises a mixer. The mixer may be configured to combine a first mixer input signal and a second mixer input signal. The mixer may provide a mixed signal. The first mixer input signal may be based on the first audio input signal. The second mixer input signal may be based on the second audio input signal. The auditory output signal may be based on the mixed signal.

An advantage of the present disclosure is that the auditory output sound may be based on a mixed signal, wherein the mixed signal may be a mixture based on audio from a sound environment, and audio from an auxiliary device and thereby improve the user experience as the hearing aid user is not limited to one audio source.

A mixer may be configured to combine the first mixer input signal and the second mixer input signal by mixing. The mixing may be a linear combination. The mixer may use a mixer parameter to weigh the first mixer input signal and the second mixer input signal. The mixed signal may be determined as

$$y_{\mathrm{mix}} = \alpha y_1 + (1 - a)y_2,$$

where $y_{\mathrm{mix}}$ is the mixed signal, $y_1$ is the first mixer input signal, $y_2$ is the second mixer input signal, and $\alpha$ is the mixer parameter. The mixer parameter may be a value between '0' and '1' including '0' and '1'. If the mixer parameter is '1', then the mixed signal is $y_1$. If the mixer parameter is '0', then the mixed signal is $y_2$. If the mixer parameter is a value between '0' and '1', the mixed signal is a combination of $y_1$ and $y_2$. The mixer parameter may be based on the input control signal.

The first mixer input signal may be the first audio input signal. The first mixer input signal may be the speech enhanced signal. The second mixer input signal may be the second audio input signal or pre-processed second audio input signal.

The mixed signal may be a combination between the speech enhanced signal and a pre-processed second audio input signal. The processed audio signal may be the mixed signal, or a post-processed processed audio signal based on the processed audio signal. The auditory output sound may be the mixed signal.

In an aspect of the present disclosure, a hearing aid system comprises an input unit. The hearing aid system comprises an auxiliary device. The auxiliary device is configured to receive a multimedia sound. The auxiliary device may provide, based on the multimedia sound, the audio for the input unit. The input unit is configured to provide an audio input signal based on the audio.

The hearing aid system comprises a signal processor. The signal processor is configured to receive the audio input signal and to provide, based on the audio input signal, a processed audio signal indicative of the multimedia sound. The signal processor is configured to provide, based on the audio input signal, an audio encoded signal. The audio encoded signal is indicative of audio characteristics in the audio.

The hearing aid system comprises a haptic unit. The haptic unit is configured to receive the audio encoded signal. The haptic unit is configured to provide, based on the audio encoded signal, a haptic stimulation. The haptic stimulation is indicative of audio in the multimedia sound.

The audio encoded signal may comprise an audio envelope signal. The audio encoded signal may comprise an audio timing signal. The audio encoded signal may comprise an audio intensity signal. The audio encoded signal may comprise a combination of the any of the aforementioned audio encoded signals.

An advantage of the present disclosure is that the haptic stimulation is based on an audio characteristic instead of the processed audio signal, where the processed audio signal may contain cues that are not relevant for haptic stimulation. Thereby, the haptic stimulation is based on relevant audio characteristics for haptic presentation in order to enhance the audio perception.

An audio envelope signal may be the envelope of a multimedia sound constituting the audio input signal. The multimedia sound may be audio from a television. The multimedia sound may be from a phone. The multimedia sound may be sound from a music player. The multimedia sound may be sound from streaming. The multimedia sound may be sound from a telecoil. The audio envelope signal may be determined based on the processed audio signal. The audio envelope signal may be determined based on an analytic signal wherein the analytic signal is based on the audio input signal. The analytic signal may be defined based on the Hilbert transform. The audio envelope signal may be determined based on average power of audio input signal in time-segments or frames, e.g. by means of the root-mean-square (RMS) value. The audio envelope signal may be determined based on the processed audio signal.

An audio timing signal may be the onset of an audio signal constituting the audio input signal. The audio timing signal may be the onset of a transient sound or an accent in the multimedia sound. The audio timing signal may be the offset of a transient sound or an accent in the multimedia sound. The onset may be defined as a timestamp wherein the energy of the multimedia sound exceeds a pre-defined or adaptive threshold. The offset may be defined as a timestamp wherein the energy of the multimedia sound is below to a pre-defined or adaptive threshold. The audio timing signal may be duration of a transient sound or an accent in the multimedia sound. The audio timing signal may be a plurality of onsets and/or offsets or durations based on a multimedia sound. The audio timing signal may be determined based on the processed audio signal.

An audio intensity signal may be the intensity or loudness of a multimedia sound constituting the audio input signal over time. The intensity may be the power or the energy of the multimedia sound. The intensity may be the dynamic range of the multimedia sound. The audio intensity signal may be determined based on the processed audio signal. The audio intensity signal may be a spectrum or part of a spectrum of the audio signal. The part of the spectrum of the audio signal may be a fundamental frequency such as an F0-component of a multimedia sound or a pitch.

The multimedia sound may be based on the processed audio signal.

The audio encoded signal may be a combination of one or more of following: the audio envelope signal, the audio timing signal, and the audio intensity signal. The combination may be determined as a linear combination between the audio encoded signals, wherein the weights may be pre-determined by a hearing care processional configuring the hearing aid system or a developer using an embodiment of the present disclosure.

The downsampler may be configured to provide the downsampled speech encoded signal based on the audio encoded signal. The downsampler may be configured to provide the downsampled speech encoded signal based on the speech encoded signal or the audio encoded signal.

The signal processor may operate in a third mode of operation. The signal processor may operate in a first mode of operation, or a second mode of operation, or a third mode of operation. In the third mode of operation, the signal processor may provide the audio encoded signal. The signal processor may comprise a switch-case configured to determine if the signal processor operates in the first or second mode of operation or the third mode of operation. The signal processor may comprise a sound controller configured to provide a sound controller input signal. The sound controller input signal may be determined by the hearing aid user through a user-interface. The sound controller input signal may be determined by the auxiliary device.

The wave modulator may comprise an amplitude modulator. The wave modulator may comprise a frequency modulator. The wave modulator may comprise an amplitude modulator and a frequency modulator.

The plurality of carrier signals may comprise a periodic pulse. The plurality of carrier signals may comprise a sinusoidal wave. The plurality of carrier signals may comprise a rectangular wave.

The plurality of carrier signals may be based on a pulse signal.

The speech encoded signal may comprise an onset signal. The speech encoded signal may comprise an offset signal. The speech encoded signal may comprise an envelope signal. The speech encoded signal may comprise a pitch signal. The speech encoded signal may comprise an intensity signal. The speech encoded signal may comprise a duration. The speech encoded signal may comprise a spectrum. The speech encoded signal may be one or more of the aforementioned speech encoded signals.

The output unit may comprise a hearing aid receiver comprising the haptic unit.

The hearing aid system may comprise a hearing aid. The hearing aid may comprise the input unit, and the signal processor, and the output unit, and the haptic unit.

The timing value may be based on a round-trip time (i.e. round-trip delay) between the the haptic device and the hearing aid. The timing value may be a signal delay between the the haptic device and the hearing aid.

The haptic device may be configured to compare the timing value with a timing threshold. The haptic device may be configured to provide a timing control signal. If the timing value is equal or below the timing threshold the haptic device may be configured to use a first haptic mode of operation.

The haptic device may be configured to provide the haptic stimulation in the first haptic mode of operation.

The haptic device may be configured to compare the timing value with a timing threshold. The haptic device may be configured to provide a timing control signal. If the timing value is above the timing threshold, the haptic device may be configured to use a second haptic mode of operation.

The haptic device may be configured to not provide the haptic stimulation in the second haptic mode of operation.

The signal processor may comprise an own voice detector. The own voice detector may be configured to receive an own voice detector input signal based on the audio input signal. The own voice detector may be configured to provide an own voice detector control signal indicative of own voice presence. The speech encoder may switch to the second mode of operation from the first mode of operation if the speech quality value is below the first threshold. The speech encoder may switch to the second mode of operation from the first mode of operation if the own voice detector control signal is indicative of own voice presence.

In a further aspect, a hearing system comprising a hearing aid as described above, in the 'detailed description of embodiments', and in the claims, AND an auxiliary device is moreover provided.

The hearing system may be adapted to establish a communication link between the hearing aid and the auxiliary device to provide that information (e.g. control and status signals, possibly audio signals) can be exchanged or forwarded from one to the other.

The auxiliary device may be constituted by or comprise a remote control, a smartphone, or other portable or wearable electronic device, such as a smartwatch or the like.

The auxiliary device may be constituted by or comprise a remote control for controlling functionality and operation of the hearing aid(s). The function of a remote control may be implemented in a smartphone, the smartphone possibly running an APP allowing to control the functionality of the audio processing device via the smartphone (the hearing aid(s) comprising an appropriate wireless interface to the smartphone, e.g. based on Bluetooth or some other standardized or proprietary scheme).

The auxiliary device may be constituted by or comprise an audio gateway device adapted for receiving a multitude of audio signals (e.g. from an entertainment device, e.g. a TV or a music player, a telephone apparatus, e.g. a mobile telephone or a computer, e.g. a PC, a wireless microphone, etc.) and adapted for selecting and/or combining an appropriate one of the received audio signals (or combination of signals) for transmission to the hearing aid.

The auxiliary device may be constituted by or comprise another hearing aid. The hearing system may comprise two hearing aids adapted to implement a binaural hearing system, e.g. a binaural hearing aid system.

A hearing aid:

The hearing aid may be adapted to provide a frequency dependent gain and/or a level dependent compression and/or a transposition (with or without frequency compression) of one or more frequency ranges to one or more other frequency ranges, e.g. to compensate for a hearing impairment of a user. The hearing aid may comprise a signal processor for enhancing the input signals and providing a processed output signal.

The hearing aid may comprise an output unit for providing a stimulus perceived by the user as an acoustic signal based on a processed electric signal. The output unit may a vibrator of a bone conducting hearing aid. The output unit may comprise an output transducer. The output transducer may comprise a receiver (loudspeaker) for providing the stimulus as an acoustic signal to the user (e.g. in an acoustic (air conduction based) hearing aid). The output transducer may comprise a vibrator for providing the stimulus as mechanical vibration of a skull bone to the user (e.g. in a bone-attached or bone-anchored hearing aid). The output unit may (additionally or alternatively) comprise a (e.g. wireless) transmitter for transmitting sound picked up-by the hearing aid to another device, e.g. a far-end communication partner (e.g. via a network, e.g. in a telephone mode of operation).

The hearing aid may comprise an input unit for providing an electric input signal representing sound. The input unit may comprise an input transducer, e.g. a microphone, for converting an input sound to an electric input signal. The input unit may comprise a wireless receiver for receiving a wireless signal comprising or representing sound and for providing an electric input signal representing said sound.

The wireless receiver and/or transmitter may e.g. be configured to receive and/or transmit an electromagnetic signal in the radio frequency range (3 kHz to 300 GHz). The wireless receiver and/or transmitter may e.g. be configured to receive and/or transmit an electromagnetic signal in a frequency range of light (e.g. infrared light 300 GHz to 430 THz, or visible light, e.g. 430 THz to 770 THz).

The hearing aid may comprise a directional microphone system adapted to spatially filter sounds from the environment, and thereby enhance a target acoustic source among a multitude of acoustic sources in the local environment of the user wearing the hearing aid. The directional system may be adapted to detect (such as adaptively detect) from which direction a particular part of the microphone signal originates. This can be achieved in various different ways as e.g. described in the prior art. In hearing aids, a microphone array beamformer is often used for spatially attenuating background noise sources. The beamformer may comprise a linear constraint minimum variance (LCMV) beamformer. Many beamformer variants can be found in literature. The minimum variance distortion-less response (MVDR) beamformer is widely used in microphone array signal processing. Ideally the MVDR beamformer keeps the signals from the target direction (also referred to as the look direction) unchanged, while attenuating sound signals from other directions maximally. The generalized sidelobe canceller (GSC) structure is an equivalent representation of the MVDR beamformer offering computational and numerical advantages over a direct implementation in its original form.

Most sound signal sources (except the user's own voice) are located far way from the user compared to dimensions of the hearing aid, e.g. a distance $d_{mic}$ between two microphones of a directional system. A typical microphone distance in a hearing aid is of the order 10 mm. A *minimum* distance of a sound source of interest to the user (e.g. sound from the user's mouth or sound from an audio delivery device) is of the order of 0.1 m (> 10 $d_{mic}$). For such minimum distances, the hearing aid (microphones) would be in the acoustic near-field of the sound source and a difference in level of the sound signals impinging on respective microphones may be significant. A *typical* distance for a communication partner is more than 1 m (>100 $d_{mic}$). The hearing aid (microphones) would be in the acoustic far-field of the sound source and a difference in level of the sound signals impinging on respective microphones is insignificant. The difference in *time of arrival* of sound impinging in the direction of the microphone axis (e.g. the front or back of a normal hearing aid) is $\Delta T = d_{mic}/v_{sound} = 0.01/343$ [s] $= 29\ \mu s$, where $v_{sound}$ is the speed of sound in air at 20°C (343 m/s).

The hearing aid may comprise antenna and transceiver circuitry allowing a wireless link to an entertainment device (e.g. a TV-set), a communication device (e.g. a telephone), a wireless microphone, a separate (external) processing device, or another hearing aid, etc. The hearing aid may thus be configured to wirelessly receive a direct electric input signal from another device. Likewise, the hearing aid may be configured to wirelessly transmit a direct electric output signal to another device. The direct electric input or output signal may represent or comprise an audio signal and/or a control signal and/or an information signal.

In general, a wireless link established by antenna and transceiver circuitry of the hearing aid can be of any type. The wireless link may be a link based on near-field communication, e.g. an inductive link based on an inductive coupling between antenna coils of transmitter and receiver parts. The wireless link may be based on far-field, electromagnetic radiation. Preferably, frequencies used to establish a communication link between the hearing aid and the other device is below 70 GHz, e.g. located in a range from 50 MHz to 70 GHz, e.g. above 300 MHz, e.g. in an ISM range above 300 MHz, e.g. in the 900 MHz range or in the 2.4 GHz range or in the 5.8 GHz range or in the 60 GHz range (ISM=Industrial, Scientific and Medical, such standardized ranges being e.g. defined by the International Telecommunication Union, ITU). The wireless link may be based on a standardized or proprietary technology. The wireless link may be based on Bluetooth technology (e.g. Bluetooth Low-Energy technology, e.g. LE audio), or Ultra WideBand (UWB) technology.

The hearing aid may be constituted by or form part of a portable (i.e. configured to be wearable) device, e.g. a device comprising a local energy source, e.g. a battery, e.g. a rechargeable battery. The hearing aid may e.g. be a low weight, easily wearable, device, e.g. having a total weight less than 100 g, such as less than 20 g, such as less than 5 g.

The hearing aid may comprise a 'forward' (or 'signal') path for processing an audio signal between an input and an output of the hearing aid. A signal processor may be located in the forward path. The signal processor may be adapted to provide a frequency dependent gain according to a user's particular needs (e.g. hearing impairment). The hearing aid may comprise an 'analysis' path comprising functional components for analyzing signals and/or controlling processing of the forward path. Some or all signal processing of the analysis path and/or the forward path may be conducted in the frequency domain, in which case the hearing aid comprises appropriate analysis and synthesis filter banks. Some or all signal processing of the analysis path and/or the forward path may be conducted in the time domain.

An analogue electric signal representing an acoustic signal may be converted to a digital audio signal in an analogue-to-digital (AD) conversion process, where the analogue signal is sampled with a predefined sampling frequency or rate $f_s$, $f_s$ being e.g. in the range from 8 kHz to 48 kHz (adapted to the particular needs of the application) to provide digital samples $x_n$ (or $x[n]$) at discrete points in time $t_n$ (or $n$), each audio sample representing the value of the acoustic signal at $t_n$ by a predefined number $N_b$ of bits, $N_b$ being e.g. in the range from 1 to 48 bits, e.g. 24 bits. Each audio sample is hence quantized using $N_b$ bits (resulting in $2^{N_b}$ different possible values of the audio sample). A digital sample x has a length in time of $1/f_s$, e.g. 50 $\mu s$, for $f_s = 20$ kHz. A number of audio samples may be arranged in a time frame. A time frame may comprise 64 or 128 audio data samples. Other frame lengths may be used depending on the practical application.

The hearing aid may comprise an analogue-to-digital (AD) converter to digitize an analogue input (e.g. from an input transducer, such as a microphone) with a predefined sampling rate, e.g. 20 kHz. The hearing aids may comprise a digital-to-analogue (DA) converter to convert a digital signal to an analogue output signal, e.g. for being presented to a user via an output transducer.

The hearing aid, e.g. the input unit, and or the antenna and transceiver circuitry may comprise a transform unit for converting a time domain signal to a signal in the transform domain (e.g. frequency domain or Laplace domain, Z transform, wavelet transform, etc.). The transform unit may be constituted by or comprise a TF-conversion unit for providing a time-frequency representation of an input signal. The time-frequency representation may comprise an array or map of corresponding complex or real values of the signal in question in a particular time and frequency range. The TF conversion unit may comprise a filter bank for filtering a (time varying) input signal and providing a number of (time varying) output signals each comprising a distinct frequency range of the input signal. The TF conversion unit may comprise a Fourier transformation unit (e.g. a Discrete Fourier Transform (DFT) algorithm, or a Short Time Fourier Transform (STFT) algorithm, or similar) for converting a time variant input signal to a (time variant) signal in the (time-)frequency domain. The frequency range considered by the hearing aid from a minimum frequency $f_{min}$ to a maximum frequency $f_{max}$ may comprise a part of the typical human audible frequency range from 20 Hz to 20 kHz, e.g. a part of the range from 20 Hz to 12 kHz. Typically, a sample rate $f_s$ is larger than or equal to twice the maximum frequency $f_{max}$, $f_s \geq 2f_{max}$. A signal of the forward and/or analysis path of the hearing aid may be split into a number $NI$ of frequency bands (e.g. of uniform width), where $NI$ is e.g. larger than 5, such as larger than 10, such as larger than 50, such as larger than 100, such as larger than 500, at least some of which are processed individually. The hearing aid may be adapted to process a signal of the forward and/or analysis path in a number $NP$ of different frequency channels $(NP \leq NI)$. The frequency channels may be uniform or non-uniform in width (e.g. increasing in width with frequency), overlapping or non-overlapping.

The hearing aid may be configured to operate in different modes, e.g. a normal mode and one or more specific modes, e.g. selectable by a user, or automatically selectable. A mode of operation may be optimized to a specific acoustic situation or environment, e.g. a communication mode, such as a telephone mode. A mode of operation may include a low-power mode, where functionality of the hearing aid is reduced (e.g. to save power), e.g. to disable wireless communication, and/or to disable specific features of the hearing aid.

The hearing aid may comprise a number of detectors configured to provide status signals relating to a current physical environment of the hearing aid (e.g. the current acoustic environment), and/or to a current state of the user wearing the hearing aid, and/or to a current state or mode of operation of the hearing aid. Alternatively or additionally, one or more detectors may form part of an *external* device in communication (e.g. wirelessly) with the hearing aid. An external device may e.g. comprise another hearing aid, a remote control, and audio delivery device, a telephone (e.g. a smartphone), an external sensor, etc.

One or more of the number of detectors may operate on the full band signal (time domain). One or more of the number of detectors may operate on band split signals ((time-) frequency domain), e.g. in a limited number of frequency bands.

The number of detectors may comprise a level detector for estimating a current level of a signal of the forward path. The detector may be configured to decide whether the current level of a signal of the forward path is above or below a given (L-)threshold value. The level detector operates on the full band signal (time domain). The level detector operates on band split signals ((time-) frequency domain).

The hearing aid may comprise a voice activity detector (VAD) for estimating whether or not (or with what probability) an input signal comprises a voice signal (at a given point in time). A voice signal may in the present context be taken to include a speech signal from a human being. It may also include other forms of utterances generated by the human speech system (e.g. singing).

The voice activity detector unit may be adapted to classify a current acoustic environment of the user as a VOICE or NO-VOICE environment. This has the advantage that time segments of the electric microphone signal comprising human utterances (e.g. speech) in the user's environment can be identified, and thus separated from time segments only (or mainly) comprising other sound sources (e.g. artificially generated noise). The voice activity detector may be adapted to detect as a VOICE also the user's own voice. Alternatively, the voice activity detector may be adapted to exclude a user's own voice from the detection of a VOICE.

The hearing aid may comprise an own voice detector for estimating whether or not (or with what probability) a given input sound (e.g. a voice, e.g. speech) originates from the voice of the user of the system. A microphone system of the hearing aid may be adapted to be able to differentiate between a user's own voice and another person's voice and possibly from NON-voice sounds.

The number of detectors may comprise a movement detector, e.g. an acceleration sensor. The movement detector may be configured to detect movement of the user's facial muscles and/or bones, e.g. due to speech or chewing (e.g. jaw movement) and to provide a detector signal indicative thereof.

The hearing aid may comprise a classification unit configured to classify the current situation based on input signals from (at least some of) the detectors, and possibly other inputs as well. **In** the present context 'a current situation' may be taken to be defined by one or more of

a) the physical environment (e.g. including the current electromagnetic environment, e.g. the occurrence of electromagnetic signals (e.g. comprising audio and/or control signals) intended or not intended for reception by the hearing aid, or other properties of the current environment than acoustic);
b) the current acoustic situation (input level, feedback, etc.), and
c) the current mode or state of the user (movement, temperature, cognitive load, etc.);
d) the current mode or state of the hearing aid (program selected, time elapsed since last user interaction, etc.) and/or of another device in communication with the hearing aid.

The classification unit may be based on or comprise a neural network, e.g. a recurrent neural network, e.g. a trained neural network.

The hearing aid may comprise an acoustic (and/or mechanical) feedback control (e.g. suppression) or echo-cancelling system. Adaptive feedback cancellation has the ability to track feedback path changes over time. It is typically based on a linear time invariant filter to estimate the feedback path but its filter weights are updated over time. The filter update may be calculated using stochastic gradient algorithms, including some form of the Least Mean Square (LMS) or the Normalized LMS (NLMS) algorithms. They both have the property to minimize the error signal in the mean square sense with the NLMS additionally normalizing the filter update with respect to the squared Euclidean norm of some reference signal.

The hearing aid may further comprise other relevant functionality for the application in question, e.g. compression, noise reduction, etc.

The hearing aid may comprise a hearing instrument, e.g. a hearing instrument adapted for being located at the ear or fully or partially in the ear canal of a user.

A method:

**In** an aspect, a method for providing an auditory output sound and a haptic stimulation is further provided. The method comprises receiving, by an input unit comprising at least one microphone, an input signal. The method comprises providing, by the input unit, an audio input signal based on the input signal. The method comprises providing, by a signal processor, a processed audio signal based on the audio input signal. The method comprises providing, by a signal processor, a speech encoded signal based on the audio input signal. The method comprises providing, by an output unit, an auditory output sound based on the processed audio signal. The method comprises providing, by a haptic unit, a haptic stimulation based on the speech encoded signal.

An advantage of the present disclosure is a method for improving speech perception for a hearing aid user which is achieved by providing an auditory output sound and a haptic stimulation.

The method for providing an auditory output sound and a haptic may be used in a hearing aid system. The method for providing an auditory output sound and a haptic may be used in combination with a sound device and a haptic device. The sound device may be a headset, a speakerphone, or a loudspeaker system.

It is intended that some or all of the structural features of the device described above, in the 'detailed description of embodiments' or in the claims can be combined with embodiments of the method, when appropriately substituted by a corresponding process and vice versa. Embodiments of the method have the same advantages as the corresponding devices.

Definitions:

**In** the present context, a hearing aid, e.g. a hearing instrument, refers to a device, which is adapted to improve, augment and/or protect the hearing capability of a user by receiving acoustic signals from the user's surroundings, generating corresponding audio signals, possibly modifying the audio signals and providing the possibly modified audio signals as audible signals to at least one of the user's ears. Such audible signals may e.g. be provided in the form of acoustic signals radiated into the user's outer ears and/or acoustic signals transferred as mechanical vibrations to the user's inner ears through the bone structure of the user's head and/or through parts of the middle ear.

The hearing aid may be configured to be worn in any known way, e.g. as a unit arranged behind the ear with a tube leading radiated acoustic signals into the ear canal or with an output transducer, e.g. a loudspeaker, arranged close to or in the ear canal, as a unit entirely or partly arranged in the pinna and/or in the ear canal, as a unit, e.g. a vibrator, attached to a fixture implanted into the skull bone, etc. The hearing aid may comprise a single unit or several units communicating (e.g. acoustically, electrically or optically) with each other. The loudspeaker may be arranged in a housing together with other components of the hearing aid, or may be an external unit in itself (possibly in combination with a flexible guiding element, e.g. a dome-like element).

A hearing aid may be adapted to a particular user's needs, e.g. a hearing impairment. A configurable signal processing circuit of the hearing aid may be adapted to apply a frequency and level dependent compressive amplification of an input signal. A customized frequency and level dependent gain (amplification or compression) may be determined in a fitting process by a fitting system based on a user's hearing data, e.g. an audiogram, using a fitting rationale (e.g. adapted to speech). The frequency and level dependent gain may e.g. be embodied in processing parameters, e.g. uploaded to the hearing aid via an interface to a programming device (fitting system), and used by a processing algorithm executed by the configurable signal processing circuit of the hearing aid.

A 'hearing system' refers to a system comprising one or two hearing aids, and a 'binaural hearing system' refers to a system comprising two hearing aids and being adapted to cooperatively provide audible signals to both of the user's ears. Hearing systems or binaural hearing systems may further comprise one or more 'auxiliary devices', which communicate with the hearing aid(s) and affect and/or benefit from the function of the hearing aid(s). Such auxiliary devices may include at least one of a remote control, a remote microphone, an audio gateway device, an entertainment device, e.g. a music player, a wireless communication device, e.g. a mobile phone (such as a smartphone) or a tablet or another device, e.g. comprising a graphical interface. Hearing aids, hearing systems or binaural hearing systems may e.g. be used for compensating for a hearing-impaired person's loss of hearing capability, augmenting or protecting a normal-hearing person's hearing capability and/or conveying electronic audio signals to a person. Hearing aids or hearing systems may e.g. form part of or interact with public-address systems, active ear protection systems, handsfree telephone systems, car audio systems, entertainment (e.g. TV, music playing or karaoke) systems, teleconferencing systems, classroom amplification systems, etc.

The invention is set out in the appended set of claims.

BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

FIG. 1 schematically shows an example embodiment of a hearing aid system according to the present disclosure.

FIG. 2 schematically shows an example embodiment of a method for providing an auditory output sound and a haptic stimulation according to the present disclosure.

FIG. 3 schematically shows an example embodiment of a hearing aid system comprising a hearing aid and a haptic device according to the present disclosure.

FIG. 4 schematically shows an example embodiment of a hearing aid system comprising a hearing aid, a haptic device, and an auxiliary device according to the present disclosure.

FIG. 5 schematically shows an example embodiment of a hearing aid constituting the hearing aid system according to the present disclosure.

FIG. 6 schematically shows an example of a haptic device constituting the hearing aid system according to the present disclosure.

FIG. 7 schematically shows an example embodiment of a hearing aid system according to the present disclosure.

FIG. 8 schematically shows an example embodiment of a hearing aid system according to the present disclosure.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microelectronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

The present application relates to the field of hearing aid systems.

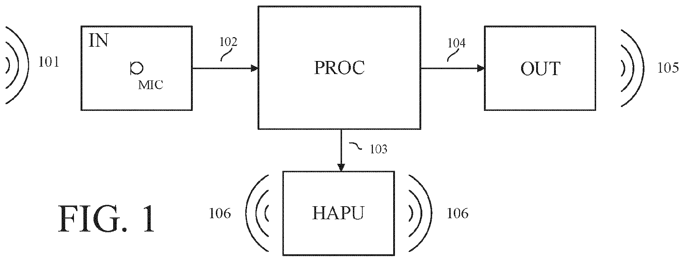

FIG. 1 shows an exemplary block diagram of an example hearing aid system according to the present disclosure. The hearing aid system comprises an input unit IN comprising at least one microphone MIC. The input unit IN is configured to pick up audio of a sound environment 101. The input unit provides at least one audio input signal 102 based on the audio. The hearing aid system comprises a signal processor PROC. The signal processor PROC is configured to provide, based on the at least one audio input signal 102, a processed output signal 104 and provide, based on the at least one audio input signal 102, a speech encoded signal 103 indicative of speech characteristics. The hearing aid system comprises a haptic unit HAPU configured to provide a haptic stimulation 104 based on the speech encoded signal 103 and indicative of speech in the sound environment. The hearing aid system comprises an output unit OUT configured to provide, based on the processed audio signal 104, an auditory output sound 105 indicative of the sound environment.

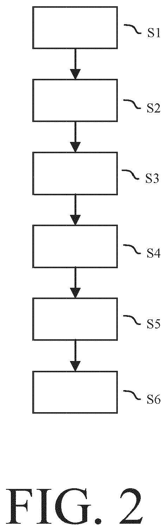

FIG. 2 shows an exemplary block diagram of an example method for providing an auditory output sound and a haptic stimulation according to the present disclosure. The method comprises receiving, by an input unit, audio in step S1. In step S2, an audio input signal is provided based on the audio, by the input unit. In step S3, a processed audio signal is provided based on the audio input signal by a signal processor. In step S4, a speech encoded signal is provided based on the audio input signal by the signal processor. In step S5, an auditory output sound is provided based on the processed audio signal by the signal processor. In step S6, a haptic stimulation is provided based on the speech encoded signal by a haptic unit.

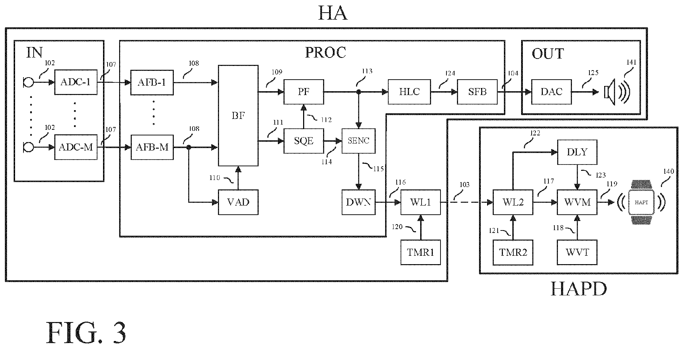

FIG. 3 shows an exemplary block diagram of a detailed example embodiment a hearing aid system comprising a hearing aid HA and a haptic device HAPD according to the present disclosure.

The hearing aid HA comprises the input unit IN, the signal processor PROC, and the output unit OUT. The haptic device HAPD comprises the haptic unit. The hearing aid HA and the haptic device HAPD are configured to wirelessly communicate 103.

The hearing aid HA comprises an input unit IN comprising a plurality of microphones configured to receive audio. The microphones are configured to provide an analog audio input signal 102.

The input unit IN comprises an analog-to-digital converter ADC-1, ..., ADC-M for each analog audio input signal 102. The analog-to-digital converters ADC-1, ..., ADC-M are configured to convert the analog audio input signals 102 to digital audio input signals 107 with a predefined sampling rate and bit-resolution. The digital input signals 107 are signals represented in the time-domain. The input unit IN provides the audio input signals 107 based on the digital audio input signals 107.

The signal processor PROC comprises an analysis filter bank AFB-1, ..., AFB-M for each audio input signal 107. The analysis filter banks AFB-1, ..., AFB-M are configured to transform the audio input signal 107 into the time-frequency domain from the time domain with a frequency band resolution K and a frame rate $F_s$. The analysis filter banks AFB-1, ..., AFB-M provide a plurality of time-frequency domain audio input signals 108.

In certain embodiments, the signal processor PROC comprises a voice activity detector (VAD) configured to detect the presence of speech per time-frequency band in the time-frequency domain audio input signal 108 and provide a voice activity detector output 110 indicative of speech characteristic (e.g. a speech activity).

The signal processor PROC can include a beamforming system BF. The beamforming system BF comprises a beamformer for each frequency band. Thus, the number of beamformers is $K$. The beamformers are adaptive beamformers configured to adapt to the background noise based on the voice activity detector output 110, such that when speech is absent, the adaptive beamformers are configured to determine noise statistics. The adaptive beamformers uses the determined noise statistics to reduce the background noise while preserving the desired speech signal when the voice activity detector VAD detects the presence of speech in the time-frequency domain audio input signal 108. The beamforming system BF is configured to provide a beamformed signal 109. The beamforming system BF is configured to provide a noise level 111 and the energy level of the beamformed signal 111 based on the determined noise statistics.

The signal processor PROC comprises a speech quality estimator SQE. The speech quality estimator is configured to determine an a-posterior signal-to-noise ratio based on the noise level 111 and the energy level of the beamformed signal 111. The speech quality estimator SQE provides the a-posterior signal-to-noise ratio 112, 114.

The signal processor PROC comprises a post-filter PF. The post-filter is a single-channel filter configured to determine, based on the a-posterior signal-to-noise ratio 112, a gain value for each frequency band. The post-filter PF is configured to apply the gain values on the beamformed signal by a multiplication for each frequency band. The post-filter PF provides a speech enhanced signal 113.

The signal processor PROC comprises a speech encoder SENC. The speech encoder SENC is configured to provide, based on the a-posterior signal-to-noise ratio and the speech enhanced signal 113, a speech encoded signal 115. The speech encoder SENC is configured to provide the speech encoded signal 115 by determining an envelope of the processed audio signal if the a-posterior signal-to-noise ratio 114 is above a first threshold (i.e. the signal processor is in a first mode of operation). The first threshold is set to 10 dB for this particular embodiment, but may easily be changed to a different value depending on a preference by the hearing aid user, or the hearing care professional, or a developer. The speech encoder SENC is configured to not provide the speech encoded signal 115 if the a-posterior signal-to-noise ratio 114 is equal or below the first threshold (i.e. the signal processor is in a second mode of operation). In this particular embodiment, the speech encoded signal may, in the second mode of operation, simply comprise values of zeros which may be regarded as equivalent to not determine the speech encoded signal 115. In the first mode of operation, the speech encoded signal 115 is determined based on first determining the absolute-square value of the speech enhanced signal 115 for each frequency band. The absolute-square value for each frequency band is then summed across frequency bands to determine a global absolute-square value. The speech encoded signal 115 is then determined by low-pass filtering the global absolute-square value.

The signal processor PROC comprises a hearing loss compensation system HLC. The hearing loss compensation system is configured to determine a compression gain in dependence of the hearing aid user's audiogram. The hearing loss compensation system is configured to provide a hearing loss compensated signal 124 based on the speech enhanced signal 113.

The signal processor PROC comprises a synthesis filter bank SFB configured to transform the hearing loss compensated signal 124 from the time-frequency domain to the time domain. The synthesis filter bank SFB provides, based on the time domain hearing aid compensated signal 124, a processed audio signal 104.

The hearing aid comprises an output unit OUT configured to receive the processed audio signal 104. The output unit OUT comprises a digital-to-analog converter DAC configured to convert the processed audio signal 104 from a digital signal to an analog signal and provide an analog processed audio signal 125. The output unit OUT comprises a loudspeaker configured to provide, based on the analog processed audio signal 125, an auditory output sound 141 being heard by the hearing aid user.

The signal processor PROC comprises a downsampler DWN configured to provide, based on the speech encoded signal, a downsampled speech encoded signal 116. The downsampled speech encoded signal 116 is determined by reducing the sample rate of the speech encoded signal 115.

The hearing aid HA comprises a first timer TMR1. The first timer TMR1 is configured to determine a first timing value 120 of the hearing aid HA. The first timing value 120 being indicative of a timestamp. The first timer is configured to provide the first timing value 120.

The hearing aid HA comprises a first wireless communication system WL1. The first wireless communication system is configured to transmit, based on the downsampled speech encoded signal 116 and first timer value 120, a wireless signal 103.

The haptic device comprises a second timer TMR2 configured to provide a second timer value 121 of the haptic device HAPD. The second timer value 121 being indicative of a timestamp.

The haptic device HAPD comprises a second wireless communication system WL2. The second wireless communication system WL2 is configured to receive the wireless signal 103, comprising the first timer value 120 and the downsampled speech encoded signal 116, and the second timer value 121. The second wireless communication system WL2 is configured to provide the downsampled speech encoded signal 117, and provide the first timer value and the second timer value 122.

The haptic device HAPD comprises a delay unit DLY configured to provide a delay value 123 based on the first timer value 120 and the second timer value 121 and a pre-defined desired bi-modal stimulation delay. The pre-defined desired bi-modal stimulation delay may be 15 ms. The delay value 123 may be determined by first subtracting the first timer value 120 from the second timer value 121 and use the intermediate result to subtract from the pre-defined desired bi-modal stimulation delay.

The haptic device HAPD comprises a wavetable WVT configured to provide a selected carrier signal 118. The wavetable WVT is configured to use a saw wave as the selected carrier signal 118.

In certain embodiments, the haptic device HAPD comprises a wave modulator WVM configured to provide a modulated signal based on the downsampled speech encoded signal 117 and the saw wave 118. The wave modulator WVM comprises a frequency modulator configured to frequency modulate the saw wave 118 and provide a frequency modulated signal. The wave modulator WVM comprises a peak detector configured to detect the peaks of the frequency modulated signal, and provide a pulse train wave based on the detected peaks. The modulated signal being the pulse train wave. The wave modulator WVM is configured to delay the modulated signal based on the delay value 123 and provide a delayed modulated signal 119.

The haptic device HAPD comprises a haptic transducer HAPT configured to provide a haptic stimulation 127 based on the delayed modulated signal 119, wherein the haptic stimulation 140 provides a physical sensation for the hearing aid user.

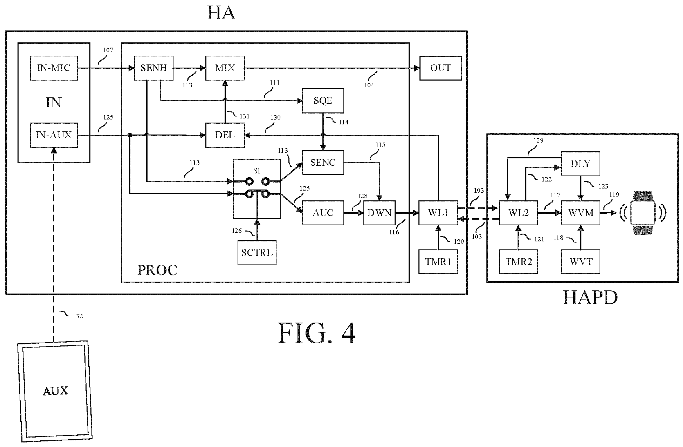

FIG. 4 shows an exemplary block diagram of a detailed example embodiment of a hearing aid system comprising a hearing aid HA, a haptic device HAPD, and an auxiliary device AUX according to the present disclosure.

The hearing aid HA comprises the input unit IN, the signal processor PROC, and the output unit OUT. The haptic device HAPD comprises the haptic unit. The hearing aid HA and the haptic device HAPD are configured to wirelessly communicate 103. The hearing aid HA and the auxiliary device AUX are configured to wirelessly communicate 132.

The hearing aid HA comprises an input unit IN comprising at least one microphone IN-MIC. The microphones IN-MIC are configured to provide a first audio input signal 107. The input unit IN comprising an aux input receiver IN-AUX configured to provide a second audio input signal 125.

The signal processor PROC comprises a speech enhancer SENH comprising a speech enhancer. The speech enhancer being configured to provide, based on the first audio input signal, a speech enhanced signal. The sound enhancer SENH is configured to provide, based on the first audio input signal 107, a speech enhanced signal 113 and a noise level 111 and the energy level of the beamformed signal 111.

The signal processor PROC comprises a speech quality estimator SQE. The speech quality estimator is configured to determine an a-posterior signal-to-noise ratio based on the noise level 111 and the energy level of the beamformed signal 111. The speech quality estimator SQE provides the a-posterior signal-to-noise ratio 114.

The signal processor PROC comprises a sound controller SCTRL configured to provide a sound controller input signal 126. The sound controller input signal 126 is determined on a hearing aid user preference through a user interface. The sound controller input signal 126 is binary.

The signal processor PROC comprises a switcher S1. The switcher S1 receives the sound controller input signal 126 and is configured to use a first program or a second program based on the sound controller input signal 126. In the first program, the signal processor PROC is configured to operate in a first mode of operation or second mode of operation. In the second program the signal processor PROC is configured to operate in a third mode of operation. In the first program, the switcher provides the speech enhanced signal 113 a speech encoder SENC and does not provide the second audio input signal 125 to an audio encoder AUC. In the second program, the switcher does not provide the speech enhanced signal 113 the speech encoder SENC and provides the second audio input signal 125 to the audio encoder AUC.

The signal processor PROC comprises a speech encoder SENC. The speech encoder SENC is configured be identical to the third embodiment in FIG. 3. The speech encoder is configured to provide the speech encoded signal 115 based on the speech enhanced signal 113 and the speech quality value 114.

The signal processor PROC comprises the audio encoder AUC. The audio encoder AUC is configured to provide, an audio envelope signal of the second audio input signal 125. The audio encoder AUC is configured to provide the audio encoded signal 128.

The signal processor PROC comprises a downsampler DWN. The downsampler DWN is configured to provide a downsampled speech encoded signal 116 if the switcher S1 is using the first program and the signal processor PROC is configured to operate in the first mode of operation. The downsampler DWN is configured to provide a downsampled audio encoded signal 116 if the switcher S1 is using the second program. The downsampled speech encoded signal 116 or the downsampled audio encoded signal 116 are determined by reducing the sample rate of the speech encoded signal 115 or the audio encoded signal 128.

The hearing aid HA comprises a first timer TMR1. The first timer TMR1 is configured to determine a first timing value 120 of the hearing aid HA. The first timing value 120 being indicative of a timestamp. The first timer is configured to provide the first timing value 120.

The hearing aid HA comprises a first wireless communication system WL1. The first wireless communication system is configured to transmit, based on the downsampled speech encoded signal 116 or the downsampled audio encoded signal 116 and first timer value 120 and a program of operation indicative if the switcher S1 is using the first program or second program, a wireless signal 103.

The haptic device comprises a second timer TMR2 configured to provide a second timer value 121 of the haptic device HAPD. The second timer value 121 being indicative of a timestamp.

The haptic device HAPD comprises a second wireless communication system WL2. The second wireless communication system WL2 is configured to receive the wireless signal 103, comprising the first timer value 120 and the downsampled speech encoded signal 116 or the downsampled audio encoded signal 116, and the second timer value 121. The second wireless communication system WL2 is configured to provide the downsampled speech encoded signal 117 or the downsampled audio encoded signal 116, and provide the first timer value and the second timer value 122.

The haptic device HAPD comprises a delay unit DLY configured to provide a delay value 123 based on the first timer value 120 and the second timer value 121 and a pre-defined desired bi-modal stimulation delay if the switcher S1 is using the first program. The pre-defined desired bi-modal stimulation delay may be 15 ms. The delay value 123 may be determined by first subtracting the first timer value 120 from the second timer value 121 and use the intermediate result to subtract from the pre-defined desired bi-modal stimulation delay.

The haptic device HAPD comprises a wavetable WVT configured to provide a selected carrier signal 118. The wavetable WVT is configured to use a saw wave as the selected carrier signal 118.

The haptic device HAPD comprises a wave modulator WVM configured to provide a modulated signal based on the downsampled speech encoded signal 117 or the downsampled audio encoded signal 117 and the saw wave 118. The wave modulator WVM comprises a frequency modulator configured to frequency modulate the saw wave 118 and provide a frequency modulated signal. The wave modulator WVM comprises a peak detector configured to detect the peaks of the frequency modulated signal, and provide a pulse train wave based on the detected peaks. The modulated signal being the pulse train wave. The wave modulator WVM is configured to delay the modulated signal based on the delay value 123 and provide a delayed modulated signal 119.

The haptic device HAPD comprises a haptic transducer HAPT configured to provide a haptic stimulation 127 based on the delayed modulated signal 119, wherein the haptic stimulation 140 provides a physical sensation for the hearing aid user.

The haptic device is configured to transmit the second timer value 121 to the hearing aid HA. The first wireless communication system WL1 receives the second timer value 121 and provides, the based on the first timer value 120 and the second timer value 121. The first wireless communication system WL1 is configured to provide a second delay value 130 if the switcher is using the second program. The second delay value 130 is determined based on a round-trip time.

The signal processor PROC comprises a delay unit DEL configured to delay, based on the second delay value 130, the second audio input signal 125 and provide a delayed second audio input signal 131.

The signal processor PROC comprises a mixer MIX configured to provide, based on the delayed second audio input signal 131 and the speech enhanced signal 113, a mixed signal 104. The mixer MIX uses a mixer parameter to weight the delayed second audio input signal 131 and the speech enhanced signal 113, and is determined by the hearing aid user.

The hearing aid HA comprises an output unit OUT configured to receive the mixed signal 104. The output unit OUT comprises a loudspeaker configured to provide, based on the mixed signal 104, an auditory output sound 141 being heard by the hearing aid user.

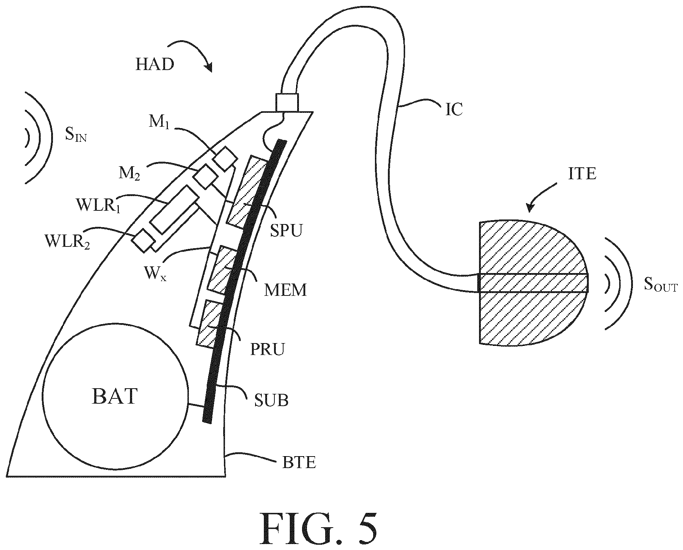

FIG. 5A shows an example embodiment of a hearing aid HA according to the present disclosure. The exemplary hearing aid HA is of a particular style (sometimes termed receiver-in-the ear, or RITE, style) comprising a BTE-part adapted for being located at or behind an ear of a user, and an ITE-part ITE adapted for being located in or at an ear canal of the user's ear and comprising a receiver (e.g. a loudspeaker). The BTE-part and the ITE-part are connected (e.g. electrically connected) by a connecting element IC and internal wiring in the ITE- and BTE-parts (cf. e.g. wiring Wx in the BTE-part). The connecting element may alternatively be fully or partially constituted by a wireless link between the BTE- and ITE-parts (or by an acoustic tube, if the loudspeaker is located in the BTE-part).

In the embodiment of a hearing aid in FIG. 5A, the BTE part comprises an input unit comprising two input transducers (e.g. microphones) $M_{BTE1}$, $M_{BTE2}$, each for providing an (first) audio input signal representative of audio originating from a sound environment $S_{IN}$ around the hearing aid. The input unit further comprises two wireless receivers $WLR_1$, $WLR_2$ (or transceivers) for providing respective directly received auxiliary audio and/or control input signals (and/or allowing transmission of audio and/or control signals to other devices, e.g. to another hearing aid, or to a remote control or auxiliary device. The hearing aid HA comprises a substrate SUB whereon a number of electronic components are mounted, including a memory MEM, e.g. storing different hearing aid programs (e.g. parameter settings defining such programs, or parameters of algorithms) and/or hearing aid configurations, e.g. input source combinations $M_{ETE1}$, $M_{BTE2}$, $WLR_1$, $WLR_2$, e.g. optimized for a number of different listening situations. In a specific mode of operation, one or more directly received auxiliary signals may be used together with one or more of the audio input signals from the microphones to provide a beamformed signal provided by applying appropriate complex weights to (at least some of) the respective signals, e.g. to provide a processed audio signal to the user (or an estimate of the user's own voice to another application, e.g. a communication partner, or a voice control interface).

The substrate SUB further comprises a configurable signal processor SPU (DSP, e.g. a digital signal processor), e.g. including a processor for applying a frequency and level dependent gain, e.g. providing hearing loss compensation, beamforming, noise reduction, filter bank functionality, and other digital functionality of a hearing aid. The configurable signal processor is adapted to access the memory (MEM. The configurable signal processor SPU is further configured to process one or more of the electric input audio signals and/or one or more of the directly received auxiliary audio input signals, based on a currently selected (activated) hearing aid program/parameter setting (e.g. either automatically selected, e.g. based on one or more sensors, or selected based on inputs from a user interface). The mentioned functional units (as well as other components) may be partitioned in circuits and components according to the application in question (e.g. with a view to size, power consumption, analogue vs. digital processing, acceptable latency, etc.), e.g. integrated in one or more integrated circuits, or as a combination of one or more integrated circuits and one or more separate electronic components (e.g. inductor, capacitor, etc.). The configurable signal processor SPU provides a processed audio signal, which is intended to be presented to a user. The substrate further comprises a front-end IC for interfacing the configurable signal processor to the input and output transducers, etc., and typically comprising interfaces between analogue and digital signals (e.g. interfaces to microphones and/or loudspeaker(s)). The input and output transducers may be individual separate components, or integrated (e.g. MEMS-based) with other electronic circuitry.

The hearing aid HA further comprises an output unit comprising an output transducer providing stimuli perceivable by the user as sound based on a processed audio signal from the processor or a signal derived therefrom. In the embodiment of a hearing aid in FIG. 5A, the ITE part comprises the output transducer in the form of a loudspeaker (also termed a 'receiver') (SPK) for converting an electric signal to an acoustic (air borne) signal, which (when the hearing aid is mounted at an ear of the user) is directed towards the ear drum (Ear drum), where sound signal $S_{OUT}$ is provided. The ITE-part further comprises a guiding element, e.g. a dome for guiding and positioning the ITE-part in the ear canal of the user. The ITE-part may further comprise a further input transducer, e.g. a microphone, facing the environment for providing an audio input signal representative of an input sound signal at the ear canal. The ITE-part may further comprise a further second input transducer, e.g. a microphone, facing the eardrum for providing a second audio input signal representative of the sound signal at the eardrum. The directly propagated sound is mixed with sound from the hearing aid to a resulting sound field at the ear drum. The sound output of the hearing aid HA may, at least in a specific mode of operation, be modified in view of the directly propagated sound from the environment to the ear drum to provide adaptive noise cancellation and/or adaptive occlusion control.

Apart from the output and input transducers, the ITE part may comprise other functional components, e.g. detectors, such as electrodes for picking up signals from the user's body such as brainwave signals, temperature indications, blood-related parameters, heartbeat indications, muscular vibrations, etc. Such detectors may include one or more of an electroencephalography (EEG) sensor, an electromyography (EMG) sensor, a movement sensor, a temperature sensor, a photoplethysmography (PPG) sensor, an electrooculography (EOG) sensor, etc.

The embodiments of a hearing aid exemplified in FIG. 5A is a portable device comprising a battery BAT, e.g. a rechargeable battery, e.g. based on Li-Ion battery technology, e.g. for energizing electronic components of the BTE- and possibly ITE-parts. In an example embodiment, the hearing device, e.g. a hearing aid, is adapted to provide a frequency dependent gain and/or a level dependent compression and/or a transposition (with or without frequency compression) of one or more frequency ranges to one or more other frequency ranges, e.g. to compensate for a hearing impairment of a user. The BTE-part may e.g. comprise a connector (e.g. a DAI or USB connector) for connecting a 'shoe' with added functionality (e.g. an FM-shoe or an extra battery, etc.), or a programming device, or a charger, or a separate processing device, etc., to the hearing aid.

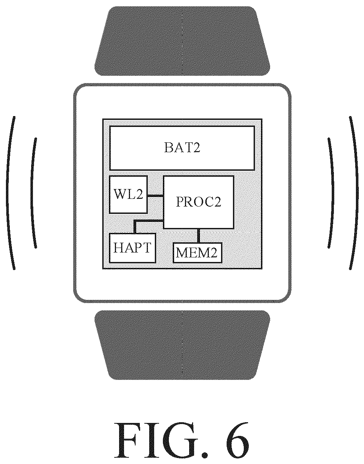

FIG. 6 schematically shows an example embodiment of a haptic device constituting a hearing aid system according to the present disclosure. The haptic device is configured to be body-worn, such as a watch. The haptic device comprises a battery unit BAT2 configured to power the electronics constituting the haptic device. The haptic device comprises a second wireless communication system WL2. The second wireless communication system WL2 is configured to receive a wireless signal from a hearing aid, wherein the wireless signal is based on a speech encoded signal. The haptic device comprises a memory unit MEM2 comprising a carrier signal. The memory unit MEM2 is configured to provide the carrier signal. The haptic device comprises a processor PROC2 configured to provide, based on the speech encoded signal and the carrier signal, a modulated signal. The haptic device comprises a haptic transducer configured to provide a haptic stimulation based on the modulated signal. The haptic stimulation being felt by the hearing aid user.

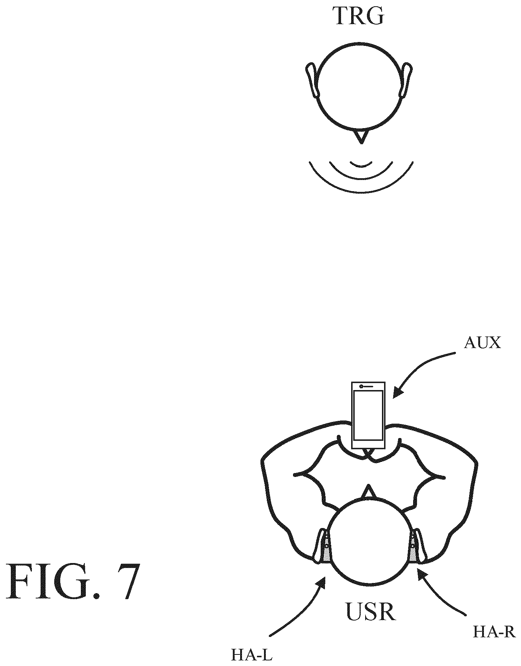

FIG. 7 schematically shows an example embodiment of a hearing aid system according to the present disclosure. The hearing aid system comprises two hearing aids HA-L, HA-R in a binaural configuration. The hearing aid system comprises a haptic device HAPD and an auxiliary device AUX. The hearing aid system is configured to pair the haptic device with the left hearing aid HA-L. The hearing aid is configured to pair the auxiliary device with the left hearing aid HA-L. In an alternative embodiment, the right hearing aid HA-R is used for pairing. In this embodiment, the auxiliary device receives a phone call and provides a second audio input signal. The left hearing aid HA-L is configured to provide, based on the second audio input signal a processed audio signal, and provide, based on the processed audio signal, an auditory output sound. The left hearing aid HA-L is configured to provide a speech encoded signal based on the second audio input signal. The haptic device is configured to provide a haptic stimulation based on the speech encoded signal.

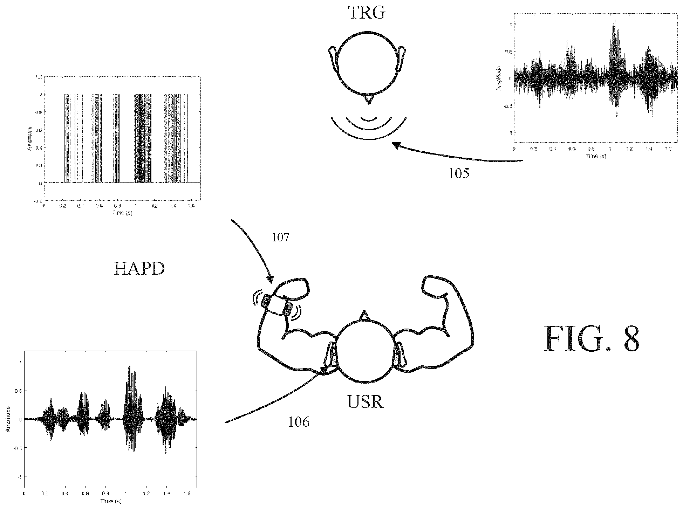

FIG. 8 schematically shows an embodiment of a hearing aid system according to the present disclosure in a second use case. The hearing aid system comprises two hearing aids HA-L, HA-R in a binaural configuration. The hearing aid system comprises a haptic device HAPD. The hearing aid system is configured to pair the haptic device with the left hearing aid HA-L. The hearing aid is configured to pair the auxiliary device with the left hearing aid HA-L. In an alternative embodiment, the right hearing aid HA-R is used for pairing. In this embodiment, the sound environment comprises a desired speaker TRG, a hearing aid user USR, and background noise. The desired speaker TRG provides a speech signal which corrupted by background noise and results in a noisy signal 105. The noisy signal 105 is picked up by microphones of the hearing aid and provides at least one audio input signal. The left hearing aid HA-L is configured to provide, based on the audio input signal, a processed audio signal 106, and provide, based on the processed audio signal 106, an auditory output sound. The left hearing aid HA-L is configured to provide a speech encoded signal based on the processed audio signal 105. The haptic device HAPD is configured to provide a haptic stimulation 107 based on the speech encoded signal.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

## ENUMERATED LIST OF ITEMS

Item 1. A hearing aid system comprising:

an input unit (IN) comprising at least one microphone (MIC) configured to pick up audio from a sound environment, wherein the input unit (IN) is configured to provide an audio input signal based on the audio; a signal processor (PROC) configured to receive the audio input signal, wherein the signal processor (PROC) is configured to provide, based on the audio input signal, a processed audio signal indicative of the audio and to provide, based on the audio input signal, a speech encoded signal indicative of speech characteristics in the audio; an output unit (OUT) configured to receive the processed audio signal and configured to provide, based on the processed audio signal, an auditory output sound indicative of the sound environment; and a haptic unit (HAPU) configured to receive the speech encoded signal and provide, based on the speech encoded signal, a haptic stimulation indicative of speech in the sound environment.

Item 2. The hearing aid system according to item 1, wherein the speech encoded signal comprises one or more of the following: a speech envelope signal, a speech timing signal, a speech intensity signal.

Item 3. The hearing aid system according to item 1 or 2, wherein the signal processor (PROC) comprises a speech enhancer (SENH) configured to provide, based on the audio input signal, a speech enhanced signal, and wherein the speech encoded signal is based on the speech enhanced signal.

Item 4. The hearing aid system according to any one of items 1-3, wherein the signal processor (PROC) comprises a downsampler (DWN) configured to reduce a data bandwidth of the speech encoded signal, and configured to provide a downsampled speech encoded signal, and wherein the speech encoded signal is based on the downsampled speech encoded signal.

Item 5. The hearing aid system according to any one of items 1-4, wherein the signal processor (PROC) comprises a speech quality estimator (SQE) configured to receive a speech quality estimator input signal based on the audio input signal, and wherein the speech quality estimator (SQE) provides a speech quality value indicative of a speech quality, and wherein the signal processor (PROC) is configured to receive the speech quality value and switch from a first mode of operation to a second mode of operation if the speech quality value is below a first threshold, and wherein the signal processor (PROC) in the first mode of operation is configured to provide the speech encoded signal, and wherein the signal processor (PROC) in the second mode of operation is configured to not provide the speech encoded signal.

Item 6. The hearing aid system according to item 5, wherein the speech quality value is indicative of one or more of following: a signal-to-noise ratio, a noise level, a speech presence probability, an own voice activity value.

Item 7. The hearing aid system according to any one of the items 1-6, wherein the haptic unit (HAPU) comprises a wave modulator (WVM) configured to receive a carrier signal and a modulator signal based on the speech encoded signal, and wherein the wave modulator (WVM) is configured to modulate the carrier signal based on the modulator signal for provision of a modulated signal, and wherein the haptic stimulation is based on the modulated signal.

Item 8. The hearing aid system according to item 7, wherein the haptic unit (HAPU) comprises a wavetable (WVT) comprising a plurality of carrier signals, and wherein the carrier signal is based on a selected carrier signal from the plurality of carrier signals of the wavetable (WVT), and wherein the haptic unit (HAPU) is configured to determine the selected carrier signal based on the speech encoded signal.

Item 9. The hearing aid system according to any one of items 1-8, wherein the input unit (IN) is configured to receive a second input signal from an auxiliary device (AUX), wherein the input unit (IN) is configured to provide a second audio input signal.

Item 10. The hearing aid system according to any one of items 1-9, wherein the hearing aid system comprises a hearing aid (HA) and a haptic device (HAPD), and wherein the hearing aid (HA) comprises the input unit (IN), the signal processor (PROC), and the output unit (OUT), and wherein the haptic device (HAPD) comprises the haptic unit (HAPU).

Item 11. The hearing aid system according to item 10, wherein the hearing aid (HA) and the haptic device (HAPD) are configured to wirelessly communicate.

Item 12. The hearing aid system according to item 10 or 11, wherein the hearing aid system is configured to determine a timing value, and wherein the hearing aid system is configured to synchronize the provision of the haptic stimulation and auditory output sound based on the timing value.

Item 13. The hearing aid system according to item 12, wherein the signal processor (PROC) comprises a delay unit (DEL) configured to apply a delay, based on the timing value, to the second audio input signal.

Item 14. The hearing aid system according to any one of item 9-13, wherein the hearing aid system comprises a mixer (MIX) configured to combine a first mixer input signal and a second mixer input signal, and provide a mixed signal, and wherein the first mixer input signal is based on the first audio input signal, and wherein the second mixer input signal is based on the second audio input signal, and wherein the auditory output signal is based on the mixed signal.

Item 15. A method for providing an auditory output sound and a haptic stimulation, the method comprises:

receiving, by an input unit (IN) comprising at least one microphone (MIC), audio;
providing, by the input unit (IN), an audio input signal based on the audio;
providing, by a signal processor (PROC), a signal processor output signal based on the audio input signal;
providing, by a signal processor (PROC), a speech encoded signal based on the audio input signal;
providing, by an output unit (OUT), an auditory output sound based on the signal processor output signal; and
providing, by a haptic unit (HAPU), a haptic stimulation based on the speech encoded signal.

Item 16. The hearing aid system according to item 7, wherein the modulator comprises an amplitude modulator and/or a frequency modulator.

Item 17. The hearing aid system according to item 8, wherein the plurality of carrier signals comprises one or more of: a periodic pulse, a sinusoidal wave, and a rectangular wave.

Item 18. The hearing aid system according to item 17, wherein the plurality of carrier signals is based on a pulse signal.

Item 19. The hearing aid system according to any one of the previous items, wherein the speech encoded signal comprises one or more of the following:

an onset signal;
an offset signal;
an envelope signal;
a pitch signal;
an intensity signal;
a duration; and
a spectrum.

Item 20. The hearing aid system according to any one of items 1-8, wherein the output unit comprises a hearing aid receiver comprising the haptic unit.

Item 21. The hearing aid system according to any one of items 1-8, wherein the hearing aid system comprises a hearing aid comprising the input unit, and the signal processor, and the output unit, and the haptic unit.

Item 22. The hearing aid system according to item 12, wherein the timing value is based on a round-trip time or a signal delay between the the haptic device and the hearing aid.

Item 23. The hearing aid system according to item 12, wherein the haptic device is configured to compare the timing value with a timing threshold and provide a timing control signal, and wherein if the timing value is equal or below the timing threshold the haptic device is configured to use a first haptic mode of operation.

Item 24. The hearing aid system according to item 23, wherein the haptic device is configured to provide the haptic stimulation in the first haptic mode of operation.

Item 25. The hearing aid system according to any one of items 22-24, wherein the haptic device is configured to compare the timing value with a timing threshold and provide a timing control signal, and wherein if the timing value is above the timing threshold the haptic device is configured to use a second haptic mode of operation.

Item 26. The hearing aid system according to item 25, wherein the haptic device is configured to not provide the haptic stimulation in the second haptic mode of operation.

Item 27. The hearing aid system according to any one of items 5-26, wherein the signal processor comprises an own voice detector configured to receive an own voice detector input signal based on the audio input signal and provide an own voice detector control signal indicative of own voice presence, and wherein the speech encoder switches to the second mode of operation from the first mode of operation if the speech quality value is below the first threshold or if the own voice detector control signal is indicative of own voice presence.

**Claims**

1. A hearing aid system comprising:

   a hearing aid (HA) comprising:

   an input unit (IN) comprising at least one microphone (MIC) configured to pick up audio from a sound environment, wherein the input unit (IN) is configured to provide an audio input signal based on the audio; a signal processor (PROC) comprising a speech enhancer (SENH) configured to attenuate noise in the audio input signal and provide, based on the audio input signal, a speech enhanced signal, and wherein the signal processor (PROC) is configured to provide a speech encoded signal based on the speech enhanced signal, and wherein the speech encoded signal comprises one or more of the following: a speech envelope signal, a speech timing signal, a speech intensity signal; an output unit (OUT) configured to provide, based on the speech enhanced signal, an auditory output sound indicative of the sound environment;

   a wrist-worn haptic device comprising an actuator configured to provide a haptic stimulation based on the speech encoded signal, and wherein the haptic stimulation is indicative of the speech enhanced signal; and wherein the hearing aid (HA) and the haptic device (HAPD) are configured to wirelessly communicate.

2. The hearing aid system according to claim 1, wherein the signal processor (PROC) comprises a downsampler (DWN) configured to reduce a data bandwidth of the speech encoded signal, and configured to provide a downsampled speech encoded signal, and wherein the speech encoded signal is based on the downsampled speech encoded signal.

3. The hearing aid system according to any one of claims 1-2, wherein the signal processor (PROC) comprises a speech quality estimator (SQE) configured to receive a speech quality estimator input signal based on the audio input signal, and wherein the speech quality estimator (SQE) provides a speech quality value indicative of a speech quality, and wherein the signal processor (PROC) is configured to receive the speech quality value and switch from a first mode of operation to a second mode of operation if the speech quality value is below a first threshold, and wherein the signal processor (PROC) in the first mode of operation is configured to provide the speech encoded signal, and wherein the signal processor (PROC) in the second mode of operation is configured to not provide the speech encoded signal.

4. The hearing aid system according to claim 3, wherein the speech quality value is indicative of one or more of following: a signal-to-noise ratio, a noise level, a speech presence probability, an own voice activity value.

5. The hearing aid system according to any one of the claims 1-4, wherein the haptic device (HAPD) comprises a wave modulator (WVM) configured to receive a carrier signal and a modulator signal based on the speech encoded signal, and wherein the wave modulator (WVM) is configured to modulate the carrier signal based on the modulator signal for provision of a modulated signal, and wherein the haptic stimulation is based on the modulated signal.

6. The hearing aid system according to claim 5, wherein the haptic device comprises a wavetable (WVT) comprising a plurality of carrier signals, and wherein the carrier signal is based on a selected carrier signal from the plurality of carrier signals of the wavetable (WVT), and wherein the haptic device (HAPD) is configured to determine the selected carrier signal based on the speech encoded signal.

7. The hearing aid system according to any one of claims 1-6, wherein the input unit (IN) is configured to receive a second input signal from an auxiliary device (AUX), wherein the input unit (IN) is configured to provide a second audio input signal.

8. The hearing aid system according to claim 7, wherein the hearing aid system comprises a mixer (MIX) configured to combine a first mixer input signal and a second mixer input signal, and provide a mixed signal, and wherein the first mixer input signal is based on the first audio input signal, and wherein the second mixer input signal is based on the second audio input signal, and wherein the auditory output signal is based on the mixed signal.

9. The hearing aid system according to any one of claims 1-8, wherein the hearing aid system is configured to determine a timing value, and wherein the hearing aid system is configured to synchronize the provision of the haptic stimulation and auditory output sound based on the timing value.

10. The hearing aid system according to claim 9 in dependence of claims 7 or 8, wherein the signal processor (PROC) comprises a delay unit (DEL) configured to apply a delay, based on the timing value, to the second audio input signal.

**11.** A method for providing an auditory output sound and a haptic stimulation, the method comprises:

receiving, by a hearing aid (HA) comprising an input unit (IN) comprising at least one microphone (MIC), audio;

providing, by the input unit (IN), an audio input signal based on the audio;

providing, by the hearing aid (HA) comprising a signal processor (PROC) comprising a speech enhancer (SENH) configured to attenuate noise in the audio input signal and provide, a speech enhanced signal based on the audio input signal,

providing, by the hearing aid (HA) comprising the signal processor (PROC), a speech encoded signal based on the speech enhanced signal, and wherein the speech encoded signal comprises one or more of the following: a speech envelope signal, a speech timing signal, a speech intensity signal;

providing, by the hearing aid (HA) comprising an output unit (OUT), an auditory output sound based on the processed audio signal; and

providing, by a wrist-worn haptic device comprising an actuator, a haptic stimulation based on the speech encoded signal, and wherein the haptic stimulation is indicative of the speech enhanced signal; and

wirelessly communicating between the hearing aid (HA) and the haptic device (HAPD).

FIG. 1

FIG. 2

FIG. 3

HA

IN
IN-MIC
SENH
MIX
OUT
IN-AUX

PROC
SQE
DEL
S1
SENC
AUC
DWN
WL1
SCTRL
TMR1

HAPD
DLY
WL2
WVM
TMR2
WVT

107 113 111 104 125 131 130 114 113 115 113 125 128 116 103 120 103 129 122 123 117 119 121 118 126 132

AUX

FIG. 4

FIG. 5

FIG. 6

TRG

AUX

FIG. 7

USR

HA-R

HA-L

TRG

105

107

HAPD

FIG. 8

USR

106

Europäisches
Patentamt

European
Patent Office

Office européen
des brevets

# EUROPEAN SEARCH REPORT

**Application Number**

EP 25 17 9875

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Mark D. Fletcher: "Using haptic stimulation to enhance auditory perception in hearing-impaired listeners", Expert Review of Medical Devices, vol. 18 29 December 2020 (2020-12-29), pages 63-74, XP002813679, Retrieved from the Internet: URL:https://www.tandfonline.com/doi/full/10.1080/17434440.2021.1863782 [retrieved on 2025-07-15] * the whole document * | 1-11 | INV. H04R25/00 A61F11/04 G09B19/04 G09B21/00 G10L21/06 G10L21/16 |
| A | Mark D. Fletcher and Carl A. Verschuur: "Electro-Haptic Stimulation: A New Approach for Improving Cochlear-Implant Listening", Front. Neurosci., vol. 15 - 2021 9 June 2021 (2021-06-09), XP002813680, Retrieved from the Internet: URL:https://www.frontiersin.org/journals/neuroscience/articles/10.3389/fnins.2021.581414/full [retrieved on 2025-07-15] * the whole document * | 1-11 | |

TECHNICAL FIELDS SEARCHED (IPC)

H04R
A61F
G09B
G10L

-/--

The present search report has been drawn up for all claims

| Place of search | Date of completion of the search | Examiner |
|---|---|---|
| Munich | 4 November 2025 | Sucher, Ralph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................................
& : member of the same patent family, corresponding document

EPO FORM 1503 03.82 (P04C01)

page 1 of 2

| | Europäisches Patentamt |
|---|---|
| | European Patent Office |
| | Office européen des brevets |

# EUROPEAN SEARCH REPORT

**Application Number**

EP 25 17 9875

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Mark D. Fletcher, Carl A. Verschuur & Samuel W. Perry: "Improving speech perception for hearing-impaired listeners using audio-to-tactile sensory substitution with multiple frequency channels", Scientific Reports, vol. 13 (2023) 16 August 2023 (2023-08-16), XP002813681, Retrieved from the Internet: URL:https://www.nature.com/articles/s41598-023-40509-7 [retrieved on 2025-07-15] * the whole document * | 1-11 | |
| A | Mark D. Fletcher, Sean R. Mills, and Tobias Goehring: "Vibro-Tactile Enhancement of Speech Intelligibility in Multi-talker Noise for Simulated Cochlear Implant Listening", Trends in Hearing, 16 September 2018 (2018-09-16), XP002813682, Retrieved from the Internet: URL:https://journals.sagepub.com/doi/full/10.1177/2331216518797838 [retrieved on 2025-07-15] * the whole document * | 1-11 | **TECHNICAL FIELDS SEARCHED (IPC)** |

The present search report has been drawn up for all claims

| Place of search | Date of completion of the search | Examiner |
|---|---|---|
| Munich | 4 November 2025 | Sucher, Ralph |

EPO FORM 1503 03.82 (P04C01)